# EUROPEAN PATENT SPECIFICATION

(11) **EP 2 901 157 B1**
(45) Date of publication and mention of the grant of the patent: **15.12.2021**
(21) Application number: 13799361.4
(22) Date of filing: 26.09.2013
(51) Int. Cl.: G01N 33/53, C07K 4/02

(54) **METHOD OF DETECTION OF SCHMALLENBERG VIRUS (SBV) AND KIT**
VERFAHREN ZUR DETEKTION DES SCHMALLENBERG-VIRUS (SBV) UND KIT
PROCÉDÉ DE DÉTECTION DE VIRUS SCHMALLENBERG (SBV) ET KIT

(30) Priority: 27.09.2012 EP 12186303; 15.08.2013 EP 13004062
(43) Date of publication of application: 05.08.2015
(73) Proprietor: IDEXX Laboratories, Inc., Westbrook, ME 04092 (US)
(72) Inventor: SCHALLER, Alain, CH-1630 Bulle (CH); SÉNÉCHAL, Yann, CH-1740 Neyruz (CH); PUN, San, CH-4125 Riehen (CH); SCHELP, Christian, CH-3110 Münsingen (CH); KRAH, Eugene, Regis, III, Yarmouth Maine (ME) 04096 (US); SIEFRING, Ann, Elizabeth, Gorham Maine (ME) 04038 (US)
(74) Representative: Helbig, Christian
(86) International application number: PCT/IB2013/002544
(87) International publication number: WO 2014/049436

(56) References cited:
- WO-A1-94/02626
- WO-A1-99/03875
- WO-A1-2013/143026
- WO-A1-2013/164476
- WO-A2-2005/038000
- WO-A2-2005/051313
- WO-A2-2013/083847
- CN-A- 1 769 294
- US-B1- 6 723 700
- US-B1- 6 830 894
- BERND HOFFMANN ET AL: "Novel Orthobunyavirus in Cattle, Europe, 2011", EMERGING INFECTIOUS DISEASES, vol. 18, no. 3, 1 March 2012 (2012-03-01), pages 469-472, XP055054135, ISSN: 1080-6040, DOI: 10.3201/eid1803.111905
- MUTIEN-MARIE GARIGLIANY ET AL: "Schmallenberg Virus in Domestic Cattle, Belgium, 2012", EMERGING INFECTIOUS DISEASES, vol. 18, no. 9, 1 January 2012 (2012-01-01), XP055053892, ISSN: 1080-6040, DOI: 10.3201/eid1809.120716
- M. F. SAEED ET AL: "Diagnosis of Oropouche Virus Infection Using a Recombinant Nucleocapsid Protein-Based Enzyme Immunoassay", JOURNAL OF CLINICAL MICROBIOLOGY, vol. 39, no. 7, 1 July 2001 (2001-07-01), pages 2445-2452, XP055053879, ISSN: 0095-1137, DOI: 10.1128/JCM.39.7.2445-2452.2001
- EMMANUEL BRÉARD ET AL: "Validation of a Commercially Available Indirect Elisa Using a Nucleocapside Recombinant Protein for Detection of Schmallenberg Virus Antibodies", PLOS ONE, vol. 8, no. 1, 15 January 2013 (2013-01-15), page e53446, XP055054132, DOI: 10.1371/journal.pone.0053446
- None

## Description

### TECHNICAL FIELD

The present disclosure relates to the cloning and expression of Schmallenberg virus (SBV) proteins or truncated proteins, peptides of SBV virus, and their use in diagnostic tests to identify an Orthobunyavirus and in particular a SBV infection in a sample, and kits for their detection.

### BACKGROUND OF THE DISCLOSURE

In 2011 cattle herds in various European countries showed an outbreak of a disease the origin and cause of which could not be linked to a known cause or disease at first.

The outbreak of this disease in European ruminants is characterized by fever, diarrhea and drop in milk yield in cattle as well as abortions, mummified fetuses, premature or stillbirths, and birth of weak or malformed lambs and calves (goat, sheep cattle).

Researchers of the Friedrich-Loeffler-Institut (FLI) were able to isolate a new virus not known so far in Europe. The isolate was identified in samples of a cattle herd in Schmallenberg, a small village in the Hochsauerland-Kreis in North Rhine-Westphalia, Germany. Accordingly, the virus was named Schmallenberg virus (SBV). The SBV was sequenced and the sequence data are accessible through the European Nucleotide Archive (https://www.ebi.ac.uk/ena/) under accession numbers HE649912, HE649913 and HE649914 (Hoffmann et al., 2012).

The virus is an enveloped, negative-sense, segmented, single-stranded RNA virus and composed of a S, M, L segment.

Bernd Hofmann et al., Emerging Infectious Diseases, vol. 18, No. 3, 1 March 2012, pages 469 - 472 describes a novel Orthobunyavirus (Schmallenberg virus) in cattle.

Mutien.Marie Garigliany et al., Emerging Infectious Diseases, vol. 18, No. 9, 1 January 2012, pages 469 - 472 describes the determination of the prevalence of antibodies against Schmallenberg virus in adult cows and proportion of infection transmitted to fetuses, tested in serum samples.

M. F. Saeed et al., Journal of Clinical Microbiology, vol. 39, No. 7, 1 July 2001, pages 2445-2452 describes the diagnosis of Oropouche virus infection using a recombinant nucleocapsid protein-based enzyme immunoassay.

Emmanuel Bréard et al., Plos One, vol. 8, No. 1, 15 January 2013, page e53446 describes the validation of a commercially available indirect ELISA using nucleocapsid recombinant protein for detection of Schmallenberg virus antibodies.

WO2013/083847 A2 describes an immunoassay leading to the rapid and simultaneous detection of antibodies to a wide range of infectious pathogens.

The above prior art documents do not disclose nor suggest the subject matter claimed herein.

Other institutes isolated viruses from brain tissue of aborted lambs in France and the Netherlands.

SBV belongs to the Orthobunyavirus genus and Bunyaviridae family and it is related to the Simbu serogroup viruses (Shamonda, Akabane, Aino virus) (Hoffmann et al., 2012; Goller et al., 2012).

Orthobunyaviruses are well known pathogens for ruminants and humans in other areas of the world like Asia, Australia, Africa and South-America. They are spread by arthropods (vector borne disease).

SBV is most probably transferred by insects e.g. midges (Culicoides spec.).

Infected premises are found all over Europe (Germany, France, Belgium, The Netherlands, UK, Italy, Luxembourg, Switzerland, and Spain). The identified cases are most probably the result of infections that took place several months ago (gestation time for cattle app. 300 days, sheep and goats app. 150 days). The disease spreads out depending on the weather conditions and activity of the vector.

The SBV can be detected in a sample by way of RT-PCT. However, the viremic phase after virus infection is very short. Thus, viral genome sequences can only be detected in the peripheral blood for a couple of days. In addition, RT-PCT is very time consuming and requires a special laboratory and trained staff to perform the analysis. Thus this test cannot be considered an easy to use diagnostic test. Another disadvantage is that RT-PCT cannot provide a high throughput analysis either. Therefore, a high throughput test of an entire herd can only be performed with a big investment in financial resources and moreover it is very time consuming. Hence it cannot serve as screening tool in the context of SBV analysis and large animal herds.

Detection of antibodies directed against SBV proteins instead of viral nucleic acids has the advantage of using the immunological memory of infected animal. Antibodies are raised against viral proteins within 10 days after infection and they can be detected over a long period of time. The existence of specific anti-SBV antibodies is an indirect proof of a SBV infection.

Recently, an indirect ELISA using as antigen recombinant SBV nucleoprotein has been put on the market by ID-Vet (167, rue Mehdi Barka, F-34070 Montpellier) under the name "ID Screen^{®} Schmallenberg virus indirect". The test is described for bovine, ovine, caprine. This test uses an indirect test format and recognizes only SBV for the indicted species.

Still there is a need to provide for a fast and reliable test for SBV infection screening world wide as an alternative and possibly with improved characteristics and applications.

Moreover, there exists the need to provide for a diagnostic test for the Simbu serogroup viruses and a pan-Simbu group test to be able to perform a fast and easy screening of herds for these infections. In view of the worldwide transport of animals a fast and reliable screening test is needed in order to make sure that infected animals are not transferred to non-infected herds. On the other hand in the course of purchasing animals the purchaser has an interest to buy healthy animals and not to learn only after having received the animals that they are infected with SBV or another Simbu serogroup virus. Such an infection will have an impact on the newborn calves and lambs and the final profit.

Hence, it is an object of the present invention to provide for a reliable SBV test, and alternatively to improve any known SBV test in order to provide for an improved and reliable SBV diagnostic test, preferably with high sensitivity and high selectivity.

It is yet another objection of the invention to provide for a validated diagnostic test for the detection of SBV.

It is yet another object of the disclosure to provide for a Simbu serogroup virus test and/or for a trans-species test.

### SUMMARY OF THE DISCLOSURE

This invention is defined by the appended claims.
I. In one aspect the disclosure relates to the propagation and purification of SBV structural and non-structural proteins, and truncated versions of these proteins, in particular the nucleocapsid (N protein) of SBV.

In yet another aspect the disclosure relates to a method of identifying if a non-human animal is infected with a Simbu serogroup virus and particularly with SBV.

In yet another aspect the disclosure relates to a kit for the detection of a Simbu serogroup virus and particularly a SBV infection in a sample.

In yet another aspect the disclosure relates to a single step test format to identify SBV in a sample, preferably or alternatively to provide for a Simbu serogroup test, preferably in a single step test format as a Simbu serogroup test.

In yet another aspect the disclosure relates to an ELISA for the detection of the Simbu serogroup virus and preferably SBV infection in a sample.

In yet another aspect the disclosure relates to a validated diagnostic test for the detection of the Simbu serogroup virus and preferably SBV infection in a sample.

In yet another aspect the disclosure relates to an assay for the detection of Bunyaviruses of veterinary importance, in particular a Simbu serogroup assay.

In yet another aspect the disclosure relates to a kit for the detection of Bunyaviruses of veterinary importance, in particular of SBV and preferably of a Simbu serogroup virus. II. In one aspect the disclosure relates to isolated peptides of the SBV nucleocapsid (N protein; Np) comprising at least one or more epitopes useful for a specific SBV or Simbu serogroup infection detection.

In another aspect the disclosure relates to a method of identifying if a non-human animal is infected with a Simbu serogroup virus and particularly with SBV.

In yet another aspect the disclosure relates to a pan-species peptide comprising an epitope useful for a method and kit for the detection of SBV and preferably the Simbu serogroup.

In yet another aspect the disclosure relates to a kit for the detection of a Simbu serogroup virus and particularly a SBV infection in a sample.

In yet another aspect the disclosure relates to a single step test format to identify SBV in a sample, preferably or alternatively to provide for a single step test format to identify Simbu serogroup virus infection.

In yet another aspect the disclosure relates to an ELISA for the detection of the Simbu serogroup virus and preferably SBV infection in a sample, preferably the sample being of cow and/or sheep origin.

In yet another aspect the disclosure relates to a validated diagnostic test for the detection of the Simbu serogroup virus, and preferably SBV infection in a sample, preferably the sample being of cow and/or sheep origin.

In yet another aspect the disclosure relates to an assay for the detection of Bunyaviruses of veterinary importance, in particular a Simbu serogroup virus assay.

In yet another aspect the disclosure relates to a kit for the detection of Bunyaviruses of veterinary importance, in particular of SBV and preferably of a Simbu serogroup virus.

### BRIEF DESCRIPTION OF THE FIGURES

### I. One aspect of the disclosure is described below.

**FIG. 1** depicts direct antigen (purified SBV nucleocapsid protein or nucleoprotein or N protein) coated to Maxisorp plates and the antigen titration in three different coating buffers tested with one positive, one negative and no sample.
**FIG. 2** depicts streptavidin pre-coated plates incubated with biotinylated N protein with decreasing protein amounts tested with three positive samples and one positive sample.
**FIG. 3** depicts the sequence of the nucleocapsid protein (N protein) of SBV (SEQ ID No. 1). **FIG. 4** depicts the amino acid sequence of the nucleocapsid protein (HisN protein) of SBV (SEQ ID No. 2) including a His tag C-terminal, preferably used in a baculovirus expression system.
**FIG. 5** depicts the nucleocapsid protein with 6x His tag amino acid sequence according to Figure 4 wherein aminoacids (aa) in bold indicate important amino acids for Simbu group viral N protein.
**FIG. 6** depicts the SBV nucleocapsid protein full length with 6xHis tag DNA sequence in an pET28a vector (SEQ ID No. 3).
**FIG. 7** depicts Nucleocapsid protein DNA sequence with consensus codons (SEQ ID No. 4). **FIG. 8** depicts Nucleocapsid truncated (protein-protein int. domain deleted): 182 aa His-tag C ― terminal (SEQ ID No. 5).

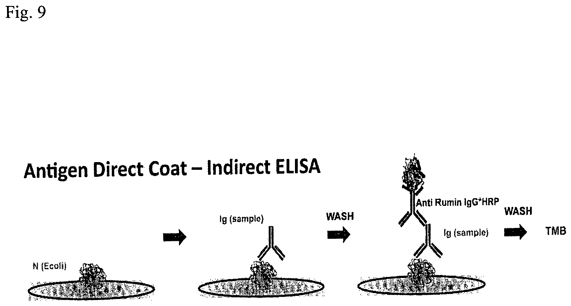

**FIG. 9** depicts an indirect ELISA format according to the disclosure.
**FIG. 10** depicts two single-step ELISA formats according to the disclosure; in the upper panel the antibody present in a positive sample is captured with one of the SBV virus proteins as described above. This protein is coated to a solid support. For detection a second antispecies specific or otherwise useful antibody is applied. This detection antibody is coupled with a detection means or can be visualized otherwise. In the particular figure the SBV capturing protein is a nucleocapsid protein as depicted in the sequence listing.

The lower panel shows a different setup of the test wherein a SBV protein is used for capturing the anti-SBV antibody in the infected sample; this protein is coated to the solid phase; the detection is performed by a second SBV antigen which is bound by the second arm of the anti-SBV antibody in the sample.

In this second variation of the disclosure of a single-step ELISA an anti-ruminant-IgG-HRPO conjugate is replaced by a SBV nucleocapsid protein coupled to HRPO in a preferred embodiment. Thus an anti-SBV antibody of the sample of the infected animal can bind with one arm the one and with its other antibody arm the other SBV protein, and can thus be detected.

### II. Another aspect of the disclosure is described in the following.

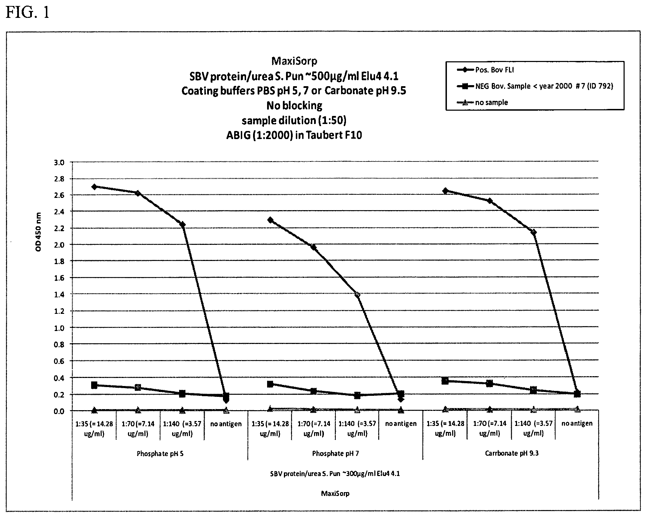

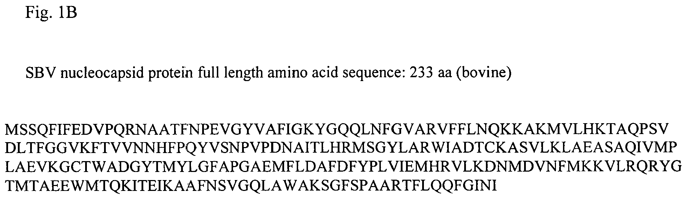

**FIG. 1B** depicts the amino acid sequence of the SBV nucleocapsid protein (N protein; Np) of SBV (SEQ ID NO: 1).

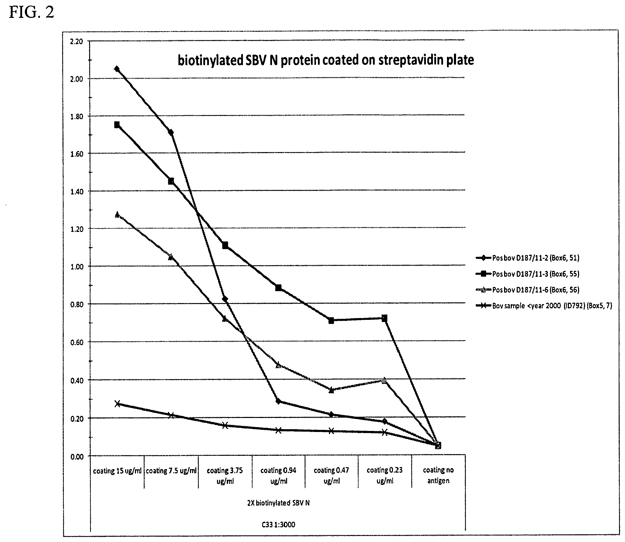

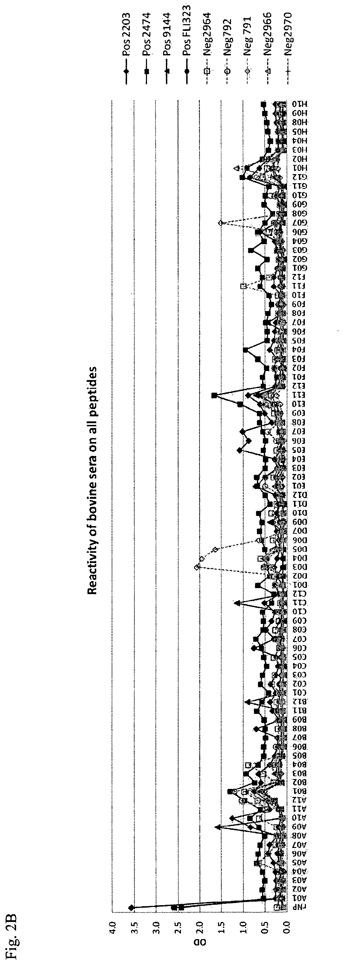

**FIG. 2B** depicts the reactivity of bovine sera on Np peptides according to the disclosure

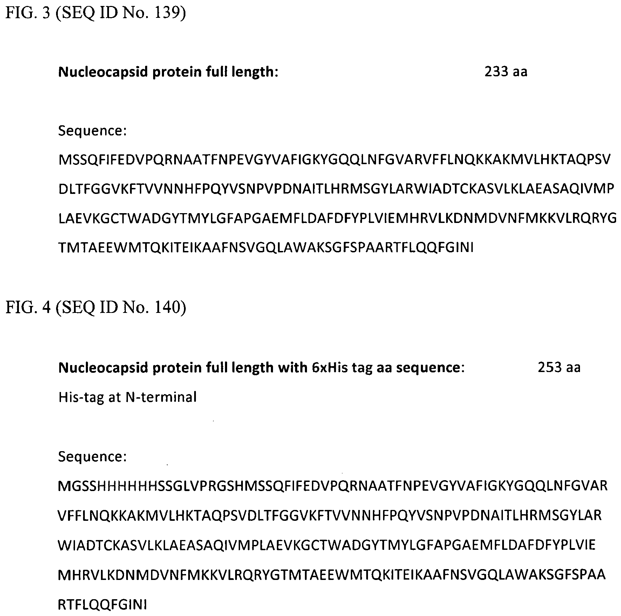

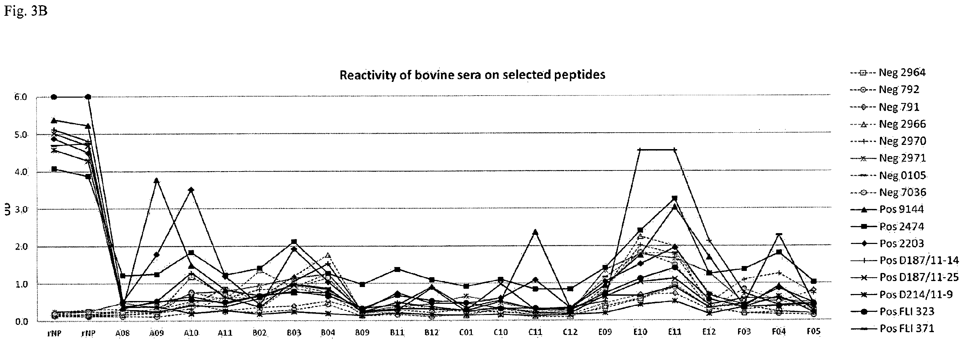

**FIG. 3B** depicts the reactivity of bovine sera on Np peptides according to the disclosure

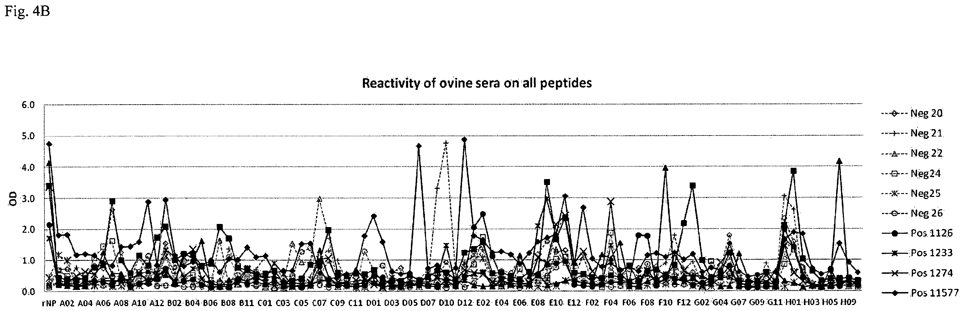

**FIG. 4B** depicts the reactivity of ovine sera on Np peptides according to the disclosure

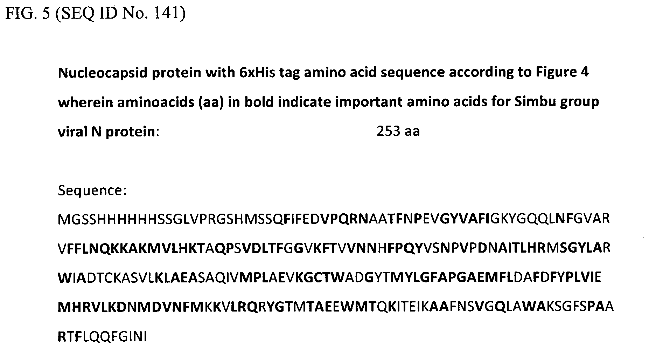

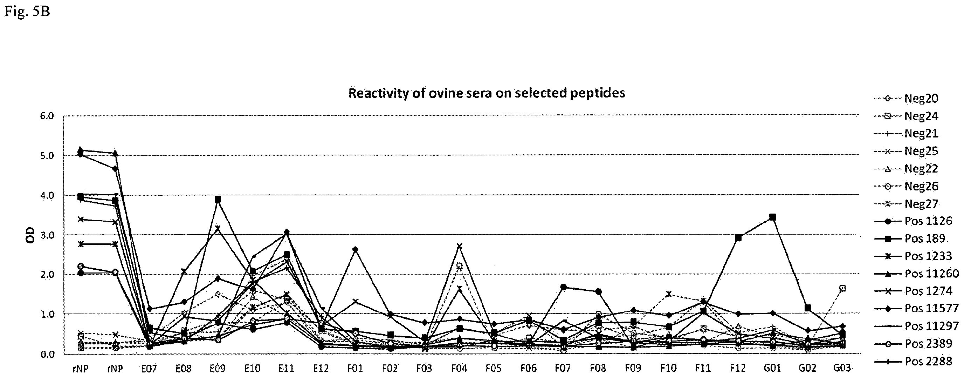

**FIG. 5B** depicts the reactivity of ovine sera on Np peptides according to the disclosure

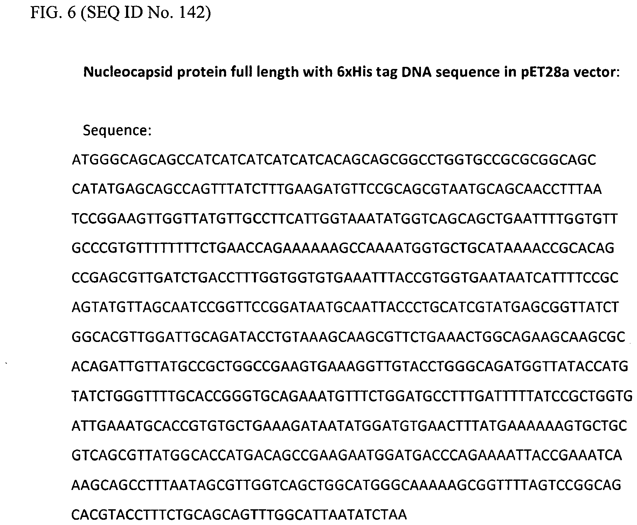

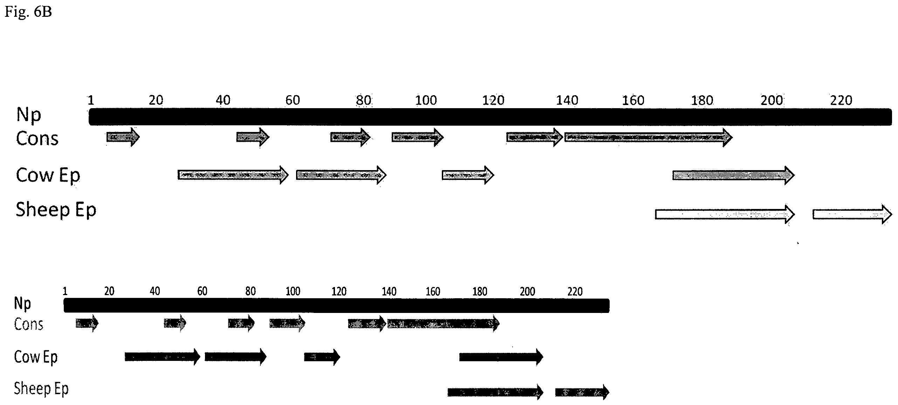

**FIG. 6B** depicts the Np amino acid sequence schematically and aligned stretches of consensus (Cons), cow epitopes of the disclosure (Cow Ep) and sheep epitopes of the disclosure (Sheep Ep). The SBV nucleoprotein (Np) amino acid sequence is represented in the first line of the Figure with Simbu serogroup highly conserved regions denoted "Cons" depicted in the second line (adapted from Savji et al., 2011 based on the information provided by Saeed et al., 2001). Regions containing preferred bovine epitopes useful for diagnostic testing are shown in the third line and denoted "Cow Ep". Regions containing preferred ovine epitopes useful for diagnostic testing are depicted in the forth line and denoted "Sheep Ep".

### DETAILED DESCRIPTION OF THE INVENTION

The invention is directed to the following aspects:
The invention is directed to an isolated peptide selected from the group consisting of the sequences VAFIGKYGQQLNFGVA, IGKYGQQLNFGVARVF, FFLNQKKAKMVLHKTA, GGVKFTVVNNHFPQYV, KFTVVNNHFPQYVSNP, WIADTCKASVLKLAEA, RVLKDNMDVNFMKKVL, KDNMDVNFMKKVLRQR, MDVNFMKKVLRQRYGT, NFMKKVLRQRYGTMTA, LRQRYGTMTAEEWMTQ, RYGTMTAEEWMTQKIT, AEEWMTQKITEIKAAF, AKSGFSPAARTFLQQF, GFSPAARTFLQQFGIN.

The invention is further directed to an isolated peptide mixture comprising at least two peptides as described above.

The invention is further directed to an in vitro method of identifying a non-human animal infected with SBV comprising the steps:
i. contacting a sample with a peptide as described above under conditions that a complex of an antibody specific against SBV in the sample and the peptide can form;
ii. detecting the thus formed complex with an antibody specific against the antibody of the complex or a labeled peptide according to any of claims 1 to 2 wherein the detection of the complex is indicative of an infection with SBV, preferably
wherein the method is an ELISA (enzyme linked immuno sorbent assay), preferably a multi-step or single-step method.

The invention is further directed to an in vitro use of one or more peptides as described above in a method for the detection of SBV infection.

The invention is further directed to a kit for the detection of a SBV infection in a sample comprising:
i. one or more peptides as described above; and
ii. a means for detection of a complex formed between the peptide and an antibody specific against SBV.

The invention is further directed to an isolated peptide as described above comprising an epitope useful for the specific identification of antibodies to SBV in a sample, wherein the epitope comprises at least 5 amino acids.

The invention is further directed to an in vitro method of identifying a non-human animal infected with SBV comprising the steps: i. contacting a sample with a peptide of SBV as described above under conditions that a complex of an antibody specific against SBV in the sample and the peptide can form; ii. detecting the thus formed complex with an antibody specific against the antibody of the complex wherein the detection of the complex is indicative of an infection with SBV, preferably wherein the method is an ELISA (enzyme linked immuno sorbent assay), preferably a multi-step or single-step method.

I. One aspect of the disclosure is described in detail below.

The various aspects of the disclosure will be described in the following in more detail.

The following definitions of important terms of the disclosure will apply throughout the specification, figures, examples and claims. Additional terms will be understood by the skilled person according to the general knowledge in the field.

As used herein, the singular forms "a," "an", and "the" include plural referents unless the context clearly dictates otherwise.

"SBV" as used herein refers to the Schmallenberg virus as isolated by the FLI (Friedrich-Loeffler-Institut) and having the sequences as described above. The SBV was sequenced and the sequence data are accessible through the European Nucleotide Archive under accession numbers HE649912, HE649913 and HE649914 (Hoffmann et al., 2012The virus is an enveloped, negative-sense, segmented, single-stranded RNA virus and composed of a S, M, L segment.

SBV belongs to the Orthobunyavirus genus and Bunyaviridae family and it is related to the Simbu serogroup viruses.

The "Simbu group viruses" or "Simbu serogroup viruses" or "pan-Simbu group viruses" as used herein comprises a group of Orthobunyaviruses which are serological related on the basis of cross-reactivity analyses (complement fixation, hemagglutination, and virus neutralization). Complement fixation activities are mediated by the viral nucleocapsid protein (N protein) while the viral glycoproteins are responsible for inducing neutralizing and hemagglutinating antibodies. Therefore a high degree of amino acid sequence homology in the N protein is one of the characteristics of the members of the Simbu serogroup. Some of the Simbu serogroup viruses are of veterinary importance and can be found in Asia, Australia, Africa, South-America and now in Europe as well. Although demarcation of Orthobunyavirus and Simbu serogroup viruses has proven difficult at least eighteen members of the serogroup including SBV are identified belonging to different virus species, e.g. the Simbu virus, Sathuperi virus, Douglas virus, Shamonda virus, Peaton virus, Sango virus, Akabane virus, Sabo virus, Tinaroo virus, Yaba-7 virus, Aino virus, Kaikalur virus, Shuni virus, Facey's Paddock virus, Oropuche virus, Utinga virus and Utive virus (Nichol et al., 2005).

"Amino acid" refers to naturally occurring and synthetic amino acids. Amino acid residues are abbreviated as follows: Alanine is A or Ala; Arginine is R or Arg; Asparagine is Nor Asn; Aspartic Acid is D or Asp; Cysteine is C or Cys; Glutamic Acid is E or Glu; Glutamine is Q or Gln; Glycine is G or Gly; Histidine is H or His; Isoleucine is I or Ile; Leucine is L or Leu; Lysine is K or Lys; Methionine is M or Met; Phenylalanine is For Phe; Proline is P or Pro; Serine is S or Ser; Threonine is T or Thr; Tryptophan is W or Trp; Tyrosine is Y or Tyr; and Valine is V or Val. Except where defined otherwise herein, X or Xaa represents any amino acid. Other relevant amino acids include, but are not limited to being, 4-hydroxyproline and 5-hydroxylysine. In all cases, the amino acid sequence of a polypeptide described or otherwise referred to herein is presented in conventional form in that the left-most, or first, amino acid residue of the sequence is the N- terminal residue and the right-most, or last, amino acid residue of the sequence is the C-terminal residue.

A "conservative variant" of any particular nucleic acid sequence includes any sequence having one or more degenerate codon substitutions to that particular nucleic acid sequence, any sequence having one or more nucleotide substitutions to, insertions to, and deletions from that particular nucleic acid sequence, and the complementary sequence of that particular nucleic acid and the conservative variants of that complementary sequence. Conservative variants of a particular nucleic acid sequence preferably have at least about 78% identity, more preferably have at least about 90% identity, and even more preferably at least about 95-99% identity, to that particular nucleic acid sequence. Conservative variants of a particular nucleic acid sequence may be artificially synthesized or they may be isolated in their natural form from an organism.

A "conservative variant" of any particular polypeptide sequence is any polypeptide having an amino acid sequence that varies from the amino acid sequence of that particular polypeptide but still retains the specific binding properties of that particular polypeptide, such that an antibody of the present disclosure that is raised against the particular polypeptide is capable of specifically binding the variant polypeptide. Therefore, for example, a conservative variant of a particular polypeptide may have one or more amino acid substitutions, deletions, additions, and insertions to that particular polypeptide. For example, a conserved variant of a particular polypeptide may have 30 or fewer, 25 or fewer, 20 or fewer, 15 or fewer, 10 or fewer, or 5 or fewer, conserved amino acid substitutions to that particular polypeptide. Conservative variants of a particular polypeptide preferably, but not essentially, have at least about 78% identity, more preferably have at least about 90% identity, and even more preferably at least about 91-99% identity, to that particular polypeptide. A percent identity for any subject nucleic acid or amino acid sequence (e.g., any of polypeptides described herein) relative to another "target" nucleic acid or amino acid sequence can be determined as follows. First, a target nucleic acid or amino acid sequence of the disclosure can be compared and aligned to a subject nucleic acid or amino acid sequence, using the BLAST 2 Sequences (B12seq) program from the stand-alone version of BLASTZ containing BLASTN and BLASTP (e.g., version 2.0.14). The stand-alone version of BLASTZ can be obtained at www.ncbi.nlm.nih.gov. Instructions explaining how to use BLASTZ, and specifically the B12seq program, can be found in the 'readme' file accompanying BLASTZ. The programms are also described in detail by Karlin et al. (1990) Proc. Natl. Acad. Sci. 87:2264;Karlin et al. (1990) Proc. Natl. Acad. Sci. 90:5873;and Altschul et al. (1997) Nucl. Acids Res. 25:3389.

As used herein, the term "polypeptide" refers to a compound of a single chain of amino acid residues linked by peptide bonds. The chain may be of any length. A protein is a polypeptide and the terms are used synonymously. The polypeptide is capable of binding one or more antibodies specific to different epitopes of the SBV polypeptides and preferably to recognize the Simbu group virus, more preferably to differentiate between the single members of said group of virus.

As used herein, the term "peptide" or "oligopeptide" refer to a single chain of amino acids consisting of between 3 and 25 amino acids, preferably of 5 to 12 amino acids.

The proteins of the present disclosure are capable of eliciting an immune response in a host animal and/or are capable of binding an antibody present in a sample of an infected animal due to an immune response after infection.

Moreover, as part of the disclosure "antibody" or "antibodies" refers to monoclonal and/or polyclonal antibodies and anti-sera which can be applied in the methods, assays and test kits of the disclosure. These antibodies function as primary or secondary detection antibodies. These antibodies may be labeled for visualization of the test result. The antibodies are applied in concentrations known by the skilled person in the context of detection methods. Preferably the antibodies are applied in a concentration of 0.1 µg/ml -5.0 µg/ml

The antibodies applied in the present disclosure may belong to any antibody class, including for example, IgG, IgM, IgA, IgD and IgE, and may be prepared by any of a variety of techniques known to the skilled artisan. (See, e.g., Dean, Methods Mol. Biol. 80:23-37 (1998); Dean, Methods Mol. Biol. 32:361-79 (1994); Baileg, Methods Mol. Biol. 32:381-88 (1994); Gullick, Methods Mol. Biol. 32:389-99 (1994); Drenckhahn et al. Alethods Cell. Biol. 37:7-56 (1993); Morrison, Ann. Rev. Immunol. 10:239-65 (1992); Wright et al. Crit. Rev. Immunol. 12:125-68(1992); Harlow and Lane, Antibodies: A Laboratory Manual, Cold Spring Harbor Laboratory (1988); and Making and Using Antibodies: A Practical Handbook, Howard and Kaser, eds., CRC Press (2006), each one of which is incorporated herein by reference in its entirety.)

An "epitope" is the antigenic determinant on a polypeptide of SBV or the Simbu group virus or one virus of this group that is recognized for binding by a paratope on antibodies specific to said SBV polypeptide. Antibodies in the context of the disclosure recognize particular epitopes having a sequence of 3 to 11, preferably 5 to 7 amino acids. The antibody may further be characterized by its binding affinity to the protein, polypeptide or peptide applied in the methods and kits of the disclosure and the binding affinity is in the range of 10 ⁻⁹ to 10⁻¹⁰ nM. Particular antibodies used in the disclosure are monoclonal or polyclonal antibodies directed against antibodies and applied as detection antibodies.

The term "label," as used herein, refers to a detectable compound, composition, or solid support, which can be conjugated directly or indirectly (e.g., via covalent or non- covalent means, alone or encapsulated) to a monoclonal antibody. The label may be detectable by itself (e.g., radioisotope labels, chemiluminescent dye, electrochemical labels, metal chelates, latex particles, or fluorescent labels) or, in the case of an enzymatic label, may catalyze chemical alteration of a substrate compound or composition which is detectable (e.g., enzymes such as horseradish peroxidase, alkaline phosphatase, and the like). The label employed in the current disclosure could be, but is not limited to alkaline phosphatase; glucose-6-phosphate dehydrogenase ("G6PDH"); horseradish peroxidase (HRP); chemiluminescers such as isoluminol, fluorescers such as fluorescein and rhodamine compounds; ribozymes; and dyes. The label may also be a specific binding molecule which itself may be detectable (e.g., biotin, avidin, streptavidin, digioxigenin, maltose, oligohistidine, 2, 4-dinitrobenzene, phenylarsenate, ssDNA, dsDNA, and the like). The utilization of a label produces a signal that may be detected by means such as detection of electromagnetic radiation or direct visualization, and that can optionally be measured.

A monoclonal antibody can be linked to a label using methods well known to those skilled in the art. E.g., Immunochemical Protocols; Methods in Molecular Biology, Vol. 295, edited by R. Bums (2005)). The detectable monoclonal antibody conjugate may be used in any known diagnostic test format like ELISA or a competitive assay format to generate a signal that is related to the presence or amount of an SBV-specific antibody in a test sample.

"Substantial binding" or "substantially bind" refer to an amount of specific binding or recognizing between molecules in an assay mixture under particular assay conditions. In its broadest aspect, substantial binding relates to the difference between a first molecule's incapability of binding or recognizing a second molecule, and the first molecules capability of binding or recognizing a third molecule, such that the difference is sufficient to allow a meaningful assay to be conducted to distinguish specific binding under a particular set of assay conditions, which includes the relative concentrations of the molecules, and the time and temperature of an incubation. In another aspect, one molecule is substantially incapable of binding or recognizing another molecule in a cross-reactivity sense where the first molecule exhibits a reactivity for a second molecule that is less than 25%, preferably less than 10%, more preferably less than 5% of the reactivity exhibited toward a third molecule under a particular set of assay conditions, which includes the relative concentration and incubation of the molecules. Specific binding can be tested using a number of widely known methods, e.g, an immunohistochemical assay, an enzyme-linked immunosorbent assay (ELISA), a radioimmunoassay (RIA), or a western blot assay.

A "biological sample" refers to a sample from an animal subject including saliva, whole blood, serum, plasma, milk or other sample known to contain anti-SBV antibodies. A biological sample can be collected and stored by any convenient means that does not impact the ability of the sample to be tested in the method of the disclosure. Samples for use in the method of the disclosure may require preparation prior to use in the method. For example, samples may need to be precipitated, diluted, filtered or centrifuged to remove unwanted components or components may be added to the sample such as salts, detergents or additional proteins.

The term "solid support" or "solid phase" refers to a non-aqueous matrix to which a SBV or a Simbu group virus polypeptide can adhere by covalent or non-covalent binding. Examples of solid support include supports formed partially or entirely of glass (e.g., controlled pore glass), synthetic and natural polymers, polysaccharides (e.g., agarose), polyacrylamides, polystyrene, polyvinyl alcohols and silicones, magnetic particles, latex particles, chromatographic strips, microtiter polystyrene plates, or any other substances that will allow bound antigens to be washed or separated from unbound materials. In certain embodiments, depending on the application, the solid support can be the well of an assay plate or can be a purification column (e.g., an affinity chromatography column).

As used herein, the term "kit" or "assay kits" (e.g., articles of manufacture) refers to an assembly of useful compounds and other means like solid support plates or test stripes for detecting SBV infection or Simbu group virus in a mammalian sample, preferably ruminants. A kit therefore may include one or more devices and/or compositions of the present disclosure. In one particular example, such a kit includes the device having an immobilized SBV protein, fragment thereof or peptide, and comprising one or more capture antibody and other useful reagents like wash reagent, as well as detector reagent and positive and negative control reagents, if desired or appropriate. Other components such as buffers, controls, and the like, known to those of ordinary skill in art, may be included in such test kits. The relative amounts of the various reagents can be varied, to provide for concentrations in solution of the reagents that substantially optimize the sensitivity of the assay. Particularly, the reagents can be provided as dry powders, usually lyophilized, which on dissolution will provide for a reagent solution having the appropriate concentrations for combining with a sample. The present kit may further include instructions for carrying out one or more methods of the present disclosure, including instructions for using any device and/or composition of the present disclosure that is included with the kit.

In the following the particular aspects of the disclosure will be described in more detail.

In one aspect of the disclosure SBV single proteins and polypeptides were expressed and purified. The full length and various truncated versions of the nucleocapsid (N) were cloned according to standard procedures known to the skilled person and produced in different expression systems. One such expression system used E.coli as is well known by the skilled person. Thus details need not be described herein and are well known from the literature. An alternative expression system that can be used is the baculovirus system also known in the art (Sambrook and Russell, 2001).

Particular details are also apparent from the Examples section.

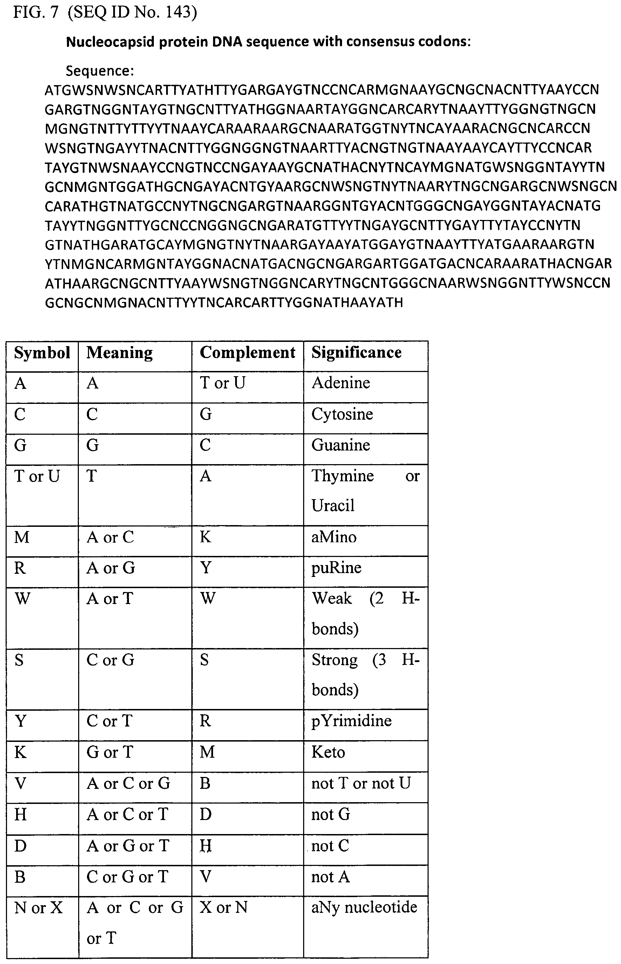

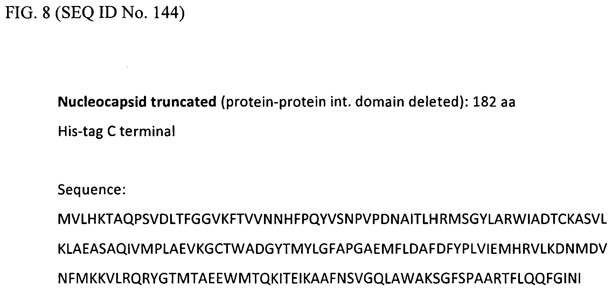

In one aspect the disclosure is an isolated SBV protein comprising or consisting of Nucleocapsid protein (N protein) having 233aa; a non-structural protein NSs (S segment) having 91 aa; a glycoprotein (Gc protein) having 846aa, preferably according to SEQ ID No. 138 to 144 (Figs. 3 to 8), or a fragment thereof, or a protein having 95% identity thereto for use in detection of SBV or a Simbu serogroup virus.

In a preferred embodiment the isolated protein is an isolated SBV peptide consisting of SEQ ID No. 139 to 144 or a peptide having 95% identity thereto.

Preferably the isolated peptide consists of an epitope useful for the specific identification of an SBV infection in a sample wherein the epitope comprises or consists of at least 3 amino acids, preferably at least 5 amino acids, more preferably at least 7 amino acids and even more preferably of 12 amino acids, even more preferably of between 5 and 25 amino acids of SEQ ID No. 139 to 144 (Figs. 3 to 8).

In another aspect the disclosure relates to a method of making a protein or polypetide as describe above.

The proteins and polypeptides of the disclosure can be cloned, expressed and purified according to methods well known in the art.

The use of different expression systems has advantages with regard to ease of the method and with regard to yield that can be achieved as well as secondary aspects like control of possible contaminations. If the proteins or peptides are applied in methods for diagnostic uses the production method may also have implications for their usefulness in the particular applications. In the current disclosure the SBV nucleocapsid (N) protein expressed in the E. coli expression system is preferred for diagnostic applications. While baculovirus expressed proteins can well be applied in diagnostic applications of the disclosure, E. coli expressed proteins are preferred.

The disclosure can use either a full length SBV N protein or truncated versions as well as such proteins which exhibit sequence modifications including but not limited to amino acid modifications, replacements, deletions, insertions or additions of sequences. Preferred embodiments are depicted in the figures and sequence listing. In a preferred embodiment the N protein as full length protein or including sequence modifications contains a C-terminal His-tag. In a preferred embodiment the N protein has a deletion of the protein-protein interaction domain and has more preferably an amino acid length of 180 to 200 amino acids, preferably the N truncated sequence has 182 amino acids. The particular sequences are depicted in the sequence listing and apparent from the Figures.

Particular SBV proteins and polypeptides can be expressed recombinantly and applied in standard diagnostic test formats well known to the skilled person. In the current disclosure an indirect ELISA format is particularly preferred, and more preferred is a single step ELISA format.

It could be shown that the SBV proteins as described above are particularly useful in tests and methods according to the disclosure to detect SBV infection, and in a more preferred embodiment Simbu serogroup viruses.

Particularly useful is the full length SBV N protein as of SEQ ID No. 139. More preferred is a truncated N protein as depicted in SEQ ID No. 144.

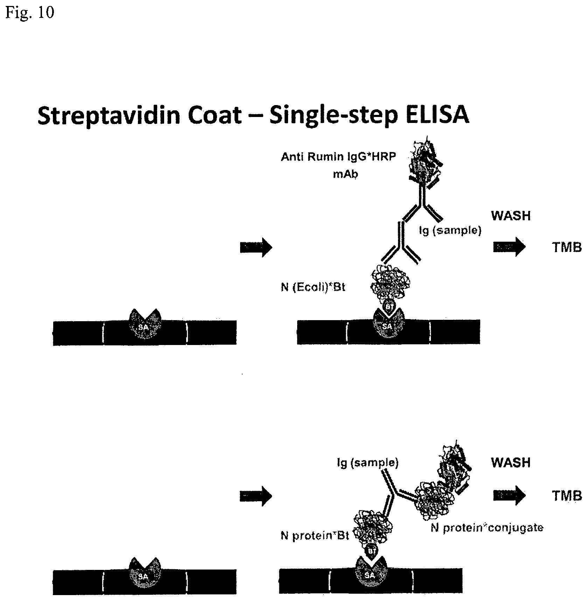

It could be shown that the application of said protein is showing particularly good results with regard to specificity and selectivity. While all known detection formats can in principal be applied for the test, certain test formats will be preferred in view of practical aspects like ease of performing them and provision of test kits. In a preferred embodiment advantageously good results were achieved in an indirect ELISA format as depicted in the Figures. More preferred is a single-step ELISA which has the advantage that all reagents and the sample can be mixed in a one-step-process. Various preferred embodiments are depicted in Fig. 10.

Further details as to the sequence and production of the proteins are depicted in the Figures and the sequence listing.

In a preferred embodiment the disclosure relates to an in vitro method of identifying a non-human animal infected with SBV or a Simbu serogroup virus comprising the steps: i. contacting a sample with a protein, polypeptide, oligopeptide or peptide of SBV under conditions that a complex of an antibody specific against SBV in the sample and the protein, polypeptide, oligopeptide or peptide of SBV can form; ii. detecting the thus formed complex with an antibody specific against the antibody of the complex wherein the detection of the complex is indicative of an infection with SBV.

In a preferred embodiment the method of identifying is an ELISA (enzyme linked immuno sorbent assay), preferably a multi-step and more preferably a single-step method.

In such a method a protein, polypeptide, oligopeptide or peptide of SBV can be applied and preferably it is selected from the group consisting of SEQ ID No. 139 to 144 (Figs. 3 to 8), or a fragment thereof.

In another aspect the disclosure relates to a kit for the detection of a SBV or Simbu serogroup virus infection in a sample comprising i. one or more proteins, polypeptides, oligopeptides or peptides of SBV as described in the Figures and sequence listing; and ii. a means for detection of a complex formed between one or more of the protein, polypeptide, oligopeptide or peptide of SBV and an antibody specific against SBV.

The protein, polypeptide, oligopeptide or peptide of SBV is preferably selected from the group consisting of SEQ ID No. 139 to 144 (Figs. 3 to 8).

In another aspect the disclosure is a method for detecting antibodies to SBV in a biological sample, the method comprising contacting the biological sample with a SBV polypeptide of SEQ ID No. 139 to 144, and detecting whether antibodies in the sample compete with the monoclonal antibody for binding to SEQ ID No. 139 to 144, thereby determining that the sample contains antibodies to SBV, wherein the SBV antibodies in a sample from animals that have been naturally infected will compete with the monoclonal antibody for binding to SEQ ID No. 139 to 144 but antibodies in a sample from animals that have not been infected or animals that have been vaccinated against SBV more than 4 weeks prior to the sample being obtained will not compete with the monoclonal antibody for binding to SEQ ID No. 139 to 144. In a preferred embodiment the method can be used for the detection of Simbu group viruses.

The disclosure achieves a sensitivity of more than 95%, preferably more than 96%, even more preferred more than 97% and a specificity of more than 97%, preferably more than 98%, and even more preferably more than 99% with the truncated N protein according to SEQ ID No. 139 to 144.

The samples that can be used in the methods, assays and test kits of the disclosure are from serum, plasma or milk preferably of ruminants and are particularly useful to detect infection in cattle, sheep or goat.

The kit may contain ready to use reagents and the test results are advantageously obtained within several hours, preferably within less than 6 hours, preferably less than 5 more preferably less than 4 hours.

The sample dilution factor is preferably less than or equal 50x, preferably 30x, preferably 20×, preferably 10× which advantageously avoids further dilution steps.

The kit preferably contains all ready to use reagents including coated plates, negative and positive controls, wash solution, sample diluent, conjugate, TMB and stop solutions.

The antigen applied is preferably an antigen which is conserved among the Simbu group viruses and thus advantageously allows a pan-Simbu group detection of infection in a sample.

In preferred embodiments the solid phase of the test is coated with antigen as described above. The antigen can be expressed in E.coli. Particularly preferred is an antigen expressed in a Baculovirus expression system. In the method and test kit any known and useful solid phase may be used, preferably MaxiSorp or PolySorp (Thermo Fisher Scientific) is used and coated by applying a coating buffer which has preferably a pH of 5, 7 pr 9.5. The antigen is applied in a quantity of 1, 2, 3 or 4 ug/ml. It may be useful to apply a blocking solution known in the art but it is preferred to avoid a blocking solution.

As sample diluent may be used 0.14M NaCl, 2.7mM KCl, Kathon 0.03%, Tween20 0.1%. Another diluent useful according to the disclosure is 2% MgCl2, 6% Tween20 and 6% AO, 0.5% Casein sodium salt

The detection antibody is diluted 1:10000, preferably 1:20000.

The method steps will be applied as required and may vary depending to the particular reagents applied. In a preferred embodiment the conditions and method steps are as follows:
Sample (1:10) in sample diluent (MgCl2 2%, AO 6%, Tween20 6%, Casein 0.5%), 100 ul/well
Incubate 1 h, room temperature in humid chamber
3x wash (phosphate buffered saline with 0.1% Tween20)
Conjugate ready-to-use, 100 µl/well
Incubate 1 h, room temperature in humid chamber
3× wash (phosphate buffered saline with 0.1% Tween20)
TMB 100ul/well, incubate 10 mns, room temperature
Add stop solution (100 µl/well) and
Read out at 450nm

In particularly preferred embodiments the sample diluent contains casein sodium salt in a concentration of between 0.1 to 0.55 %, preferably 0.1 %, more preferably 0.55 %. In preferred embodiments the sample diluent contains 0.5 % casein sodium salt and MgCl2, preferably the MgCl2 has a concentration of 2%.

In a preferred embodiment the method of detection, and preferably also the kit, is characterized by the inclusion of specific compounds, the use of particular dilutions of the capturing antigen and/or a particular amount and quality of capturing antigen coated onto the solid support used in the method of detection and the kit of the disclosure.

It could be shown that by way of the particular combination of preferably the purification conditions and/or the dilution conditions and/or the particular components included in the method of detection and kit an improved performance of the method of SBV detection and preferably of Simbu serogroup viruses could be achieved.

All members of this group could be detected with the method of the disclosure. Certain viruses are preferably detected: Schmallenberg virus, Shamonda virus, Douglas virus, Peaton virus, Akabane virus, Aino virus, Oropouche virus, Tinaroo virus, Shuni virus. Particularly preferred and particular good results were achieved with the following species: Peaton, Acabane, Aino, Tinaroo. In a particularly preferred embodiment the Simbu serogroup viruses of Bovidae, Cervidae and Camelidae are detected.

In a particularly preferred embodiment the dilution of the SBV protein, preferably the nucleocapsid protein, was chosen to be in the coating solution in a concentration of 0.5 to 5 µg/ml, preferably 1 to 3 µg/ml. The solid support to which the SBV protein is coated contains antigen from 0.25 to 1µg/well, preferably 0.5 to 1µg/well. The inventive nucleocapsid preparation in its particular dilution proofed advantageous.

Accordingly, it was achieved to have the SBV coating protein substantially in solution and the formation or presence of aggregates of the coating protein, preferably the nucleocapsid protein as described above and in SEQ ID No. 139 to 144, could be avoided. Hence, the specificity of the test could be increased and it was not evident that this aspect of the test could lead to the advantageous specificity and/or sensitivity. The positive results are evident from performing the test with confirmed positive and negative samples regarding SBV infection as such.

Moreover, the advantage of the test and kit according to the disclosure as described herein is not only evident from the positive test results achievable with the test as such. It is more so evident in comparison with test kits on the market as of today, i.e. September 2012. In particular the current disclosure and the method of detection in preferred embodiments is superior to SBV tests of the prior art, like the IDvet test and kit described above in the background section.

Another preferred embodiment uses in the method of detection and preferably in the kit is casein. Any casein available in the market can be applied in a concentration which can be adapted according to the needs of the other compounds and in order to achieve good detection results in the test. Preferably the concentration in a solution of the test is 0.1 to 1 %, preferably 0.25 to 0.75 %, more preferably 0.5 %. In a preferred embodiment the casein is present in the wash solution of the coating step wherein the antigen is coated to the solid support device.

The coating step is preferably performed at pH 5 to 10, preferably about 5, 7 or 9.5.

The coating protein, i.e. the antigen which may be preferably SBV nucleocapsid protein as described in the specification and Examples, is used in amounts of 1, 2, or 4 µg/ml, preferably 1 µg/ml.

Another preferred embodiment uses alone or in addition aminoxid (AO). Preferably the concentration used is in a solution of the method in an amount of 3 to 10%, preferably 5 to 8%, more preferably 6%.

Another preferred embodiment of the method of detection and kit contains Tween, preferably a Tween20, or a comparable substance, preferably a detergent with comparable characteristics. This substance is contained in an amount of 0.05 to 0.5%, preferably 0.1 to 0.2 %.

In a particularly preferred embodiment the wash solution of the coating step contains preferably NaCl 0.14M, KCl 2.7 mM; Kathon 0.03%, Tween20 0.1%, sample diluent comprises MgCl2 2%, aminoxid (AO) 6%, Tween20 6% and 0.5% casein.

In a preferred embodiment, the anti-ruminant conjugate is used in a dilution of 1:10 000 to 1:30 000, preferably 1:20 000 in a conjugate stabilizing buffer as a ready to use format (RTU).

In another preferred embodiment the above described compounds can be combined and will lead to particularly good test results. In such an embodiment the various compounds are contained in any of the solutions used for preparation of the kit compounds or during performing the test and may be contained in the kit according to the disclosure.

The inventors surprisingly achieved with one or a combination of the above described preferred embodiments very good results with regard to specificity and selectivity. It was not foreseeable that such good results could be obtained by using the particular antigen preparation in its particular dilution is particularly advantageous. More advantageous is the antigen dilution in connection with the other additives like casein, aminooxid and/or Tween, preferably in the concentrations as described above.

The test preferably detects the Simbu serogroup viruses, it can preferably detect SBV in bovine, goat and sheep samples.

The cut off can be chosen according to the needs of sensitivity and specificity. Distribution is regarded to adapt it to fitness for purpose. This will depend on the needs of the test. ROC is used for analysis. The cut off is chosen according to standard procedures with regard to a positive and negative controls and the respective calculations known to the person skilled in the art and literature for disgnostic test development.

In order to optimize the specificity the cut off is chosen preferably at 30% or more, 40% or more, 50% or more, or 60% or more.

In order to optimize the sensitivity the cut off is chosen preferably at 10% or more, 20% or more, 30% or more, or 40% or more.

An optimal cut off for the test according to the disclosure is preferably 40%, more preferably between 30 and 40%.

The advantage of the tests of the disclosure is that in preferred embodiments a specificity of more than 97%, preferably 98% and more preferably more than 99%, and a sensitivity of more than 94%, more preferably more than 95%, and more preferably more than 96% can be achieved. Particular embodiments achieve a specificity of about 99.8% and a sensitivity of about 95.8%. With regard to particular species the following specificities and sensitivities, respectively, can be achieved: cattle: 99.5% and 95.7%; sheep: 100% and 99%; goat: 100% and 99%.

Accordingly, the method and test kit will be particularly reliable for goat, sheep and cattle for the detection of SBV. In a preferred embodiment the good results will also apply to the

Simbu serogroup. In particular good results can be achieved to detect Akabane, Peaton, Aino and Tinaroo virus.

II. Another aspect of the disclosure is described in detail below.

The various aspects of the disclosure will be described in the following in more detail.

The following definitions of important terms of the disclosure will apply throughout the specification, figures, examples and claims. Additional terms if applicable will be understood by the skilled person according to the general knowledge in the field.

As used herein, the singular forms "a," "an", and "the" include plural referents unless the context clearly dictates otherwise.

"SBV" as used herein refers to the Schmallenberg virus as isolated by the FLI (Friedrich-Loeffler-Institut) and having the sequences as described above. The SBV was sequenced and the sequence data are accessible through the European Nucleotide Archive under accession numbers HE649912, HE649913 and HE649914 (Hoffmann et al., 2012). The virus is an enveloped, negative-sense, segmented, single-stranded RNA virus and composed of a S, M, and L segment.

SBV belongs to the Orthobunyavirus genus and Bunyaviridae family and it is related to the Simbu serogroup viruses.

The "Simbu group viruses" or "Simbu serogroup viruses" or "pan-Simbu group viruses" as used herein comprises a group of Orthobunyaviruses which are serological related on the basis of cross-reactivity analyses (complement fixation, hemagglutination, and virus neutralization). Complement fixation activities are mediated by the viral nucleocapsid protein (N protein) while the viral glycoproteins are responsible for inducing neutralizing and hemagglutinating antibodies. Therefore a high degree of amino acid sequence homology in the N protein is one of the characteristics of the members of the Simbu serogroup. Some of the Simbu serogroup viruses are of veterinary importance and can be found in Asia, Australia, Africa, South-America and now in Europe as well. Although demarcation of Orthobunyavirus and Simbu serogroup viruses has proven difficult at least eighteen members of the serogroup including SBV are identified belonging to different virus species, e.g. the Simbu virus, Sathuperi virus, Douglas virus, Shamonda virus, Peaton virus, Sango virus, Akabane virus, Sabo virus, Tinaroo virus, Yaba-7 virus, Aino virus, Kaikalur virus, Shuni virus, Facey's Paddock virus, Oropuche virus, Utinga virus and Utive virus (Nichol et al., 2005).

"Amino acid" refers to naturally occurring and synthetic amino acids. Amino acid residues are abbreviated as follows: Alanine is A or Ala; Arginine is R or Arg; Asparagine is Nor Asn; Aspartic Acid is D or Asp; Cysteine is C or Cys; Glutamic Acid is E or Glu; Glutamine is Q or Gln; Glycine is G or Gly; Histidine is H or His; Isoleucine is I or Ile; Leucine is L or Leu; Lysine is K or Lys; Methionine is M or Met; Phenylalanine is For Phe; Proline is P or Pro; Serine is S or Ser; Threonine is T or Thr; Tryptophan is W or Trp; Tyrosine is Y or Tyr; and Valine is V or Val. Except where defined otherwise herein, X or Xaa represents any amino acid. Other relevant amino acids include, but are not limited to being, 4-hydroxyproline and 5-hydroxylysine. In all cases, the amino acid sequence of a polypeptide described or otherwise referred to herein is presented in conventional form in that the left-most, or first, amino acid residue of the sequence is the N- terminal residue and the right-most, or last, amino acid residue of the sequence is the C-terminal residue.

A "conservative variant" of any particular nucleic acid sequence includes any sequence having one or more degenerate codon substitutions to that particular nucleic acid sequence, any sequence having one or more nucleotide substitutions to, insertions to, and deletions from that particular nucleic acid sequence, and the complementary sequence of that particular nucleic acid and the conservative variants of that complementary sequence. Conservative variants of a particular nucleic acid sequence preferably have at least about 78% identity, more preferably have at least about 90% identity, and even more preferably at least about 95-99% identity, to that particular nucleic acid sequence. Conservative variants of a particular nucleic acid sequence may be artificially synthesized or they may be isolated in their natural form from an organism.

A "conservative variant" of any particular polypeptide sequence is any polypeptide (peptide) having an amino acid sequence that varies from the amino acid sequence of that particular polypeptide but still retains the specific binding properties of that particular polypeptide, such that an antibody of the present disclosure that is raised against the particular polypeptide is capable of specifically binding the variant polypeptide. Therefore, for example, a conservative variant of a particular polypeptide may have one or more amino acid substitutions, deletions, additions, and insertions to that particular polypeptide. For example, a conserved variant of a particular polypeptide may have 30 or fewer, 25 or fewer, 20 or fewer, 15 or fewer, 10 or fewer, or 5 or fewer, conserved amino acid substitutions to that particular polypeptide. Conservative variants of a particular polypeptide preferably, but not essentially, have at least about 78% identity, more preferably have at least about 90% identity, and even more preferably at least about 91-99% identity, to that particular polypeptide. A percent identity for any subject nucleic acid or amino acid sequence (e.g., any of polypeptides described herein) relative to another "target" nucleic acid or amino acid sequence can be determined as follows. First, a target nucleic acid or amino acid sequence of the disclosure can be compared and aligned to a subject nucleic acid or amino acid sequence, using the BLAST 2 Sequences (B12seq) program from the stand-alone version of BLASTZ containing BLASTN and BLASTP (e.g., version 2.0.14). The stand-alone version of BLASTZ can be obtained at www.ncbi.nlm.nih.gov. Instructions explaining how to use BLASTZ, and specifically the B12seq program, can be found in the 'readme' file accompanying BLASTZ. The programs are also described in detail by Karlin et al. (1990) Proc. Natl. Acad. Sci. 87:2264; Karlin et al. (1990) Proc. Natl. Acad. Sci. 90:5873; and Altschul et al. (1997) Nucl. Acids Res. 25:3389.

As used herein, the term "peptide" refers to a single chain of amino acids consisting of between 5 and 50 amino acids, preferably of 5 to 40, more preferably 5 to 20 amino acids, more preferably 12 to 16 amino acids and more preferably 5 to 7 amino acids.

The peptides of the present disclosure are preferably capable of eliciting an immune response in a host animal and/or are capable of binding an antibody present in a sample of an infected animal due to an immune response after infection.

Moreover, as part of the disclosure "antibody" or "antibodies" refers to monoclonal and/or polyclonal antibodies and anti-sera which can be applied in the methods, assays and test kits of the disclosure. These antibodies function as primary or secondary detection antibodies. These antibodies may be labeled for visualization of the test result. The antibodies are applied in concentrations known by the skilled person in the context of detection methods. Preferably the antibodies are applied in a concentration of 0.1 µg/ml -5.0 µg/ml

The antibodies applied in the present disclosure may belong to any antibody class, including for example, IgG, IgM, IgA, IgD and IgE, and may be prepared by any of a variety of techniques known to the skilled artisan. (See, e.g., Dean, Methods Mol. Biol. 80:23-37 (1998); Dean, Methods Mol. Biol. 32:361-79 (1994); Baileg, Methods Mol. Biol. 32:381-88 (1994); Gullick, Methods Mol. Biol. 32:389-99 (1994); Drenckhahn et al. Alethods Cell. Biol. 37:7-56 (1993); Morrison, Ann. Rev. Immunol. 10:239-65 (1992); Wright et al. Crit. Rev. Immunol. 12:125-68(1992); Harlow and Lane, Antibodies: A Laboratory Manual, Cold Spring Harbor Laboratory (1988); and Making and Using Antibodies: A Practical Handbook, Howard and Kaser, eds., CRC Press (2006), each one of which is incorporated herein by reference in its entirety.)

An "epitope" is the antigenic determinant on a peptide comprising a sequence of SBV or the Simbu group virus or one virus of this group that is recognized for binding by a paratope on antibodies specific to said SBV polypeptide. Antibodies in the context of the disclosure recognize particular epitopes having a sequence of 3 to 11, preferably 5 to 7 amino acids. The antibody may further be characterized by its binding affinity to the peptide applied in the methods and kits of the disclosure and the binding affinity is in the range of 10 ⁻⁹ to 10 ⁻¹⁰ nM. Particular antibodies used in the disclosure are monoclonal or polyclonal antibodies directed against antibodies and applied as detection antibodies. "Epitope" may also be denoted immuno dominant region (IDR). In the current disclosure an "epitope" is located on a peptide according to the disclosure.

The term "label," as used herein, refers to a detectable compound, composition, or solid support, which can be conjugated directly or indirectly (e.g., via covalent or non- covalent means, alone or encapsulated) to a monoclonal antibody. The label may be detectable by itself (e.g., radioisotope labels, chemiluminescent dye, electrochemical labels, metal chelates, latex particles, or fluorescent labels) or, in the case of an enzymatic label, may catalyze chemical alteration of a substrate compound or composition which is detectable (e.g., enzymes such as horseradish peroxidase, alkaline phosphatase, and the like). The label employed in the current disclosure could be, but is not limited to alkaline phosphatase; glucose-6-phosphate dehydrogenase ("G6PDH"); horseradish peroxidase (HRP); chemiluminescers such as isoluminol, fluorescers such as fluorescein and rhodamine compounds; ribozymes; and dyes. The label may also be a specific binding molecule which itself may be detectable (e.g., biotin, avidin, streptavidin, digioxigenin, maltose, oligohistidine, 2, 4-dinitrobenzene, phenylarsenate, ssDNA, dsDNA, and the like). The utilization of a label produces a signal that may be detected by means such as detection of electromagnetic radiation or direct visualization, and that can optionally be measured.

A monoclonal antibody can be linked to a label using methods well known to those skilled in the art. E.g., Immunochemical Protocols; Methods in Molecular Biology, Vol. 295, edited by R. Bums (2005)). The detectable monoclonal antibody conjugate may be used in any known diagnostic test format like ELISA or a competitive assay format to generate a signal that is related to the presence or amount of an SBV-specific antibody in a test sample.

"Substantial binding" or "substantially bind" refer to an amount of specific binding or recognizing between molecules in an assay mixture under particular assay conditions. In its broadest aspect, substantial binding relates to the difference between a first molecule's incapability of binding or recognizing a second molecule, and the first molecules capability of binding or recognizing a third molecule, such that the difference is sufficient to allow a meaningful assay to be conducted to distinguish specific binding under a particular set of assay conditions, which includes the relative concentrations of the molecules, and the time and temperature of an incubation. In another aspect, one molecule is substantially incapable of binding or recognizing another molecule in a cross-reactivity sense where the first molecule exhibits a reactivity for a second molecule that is less than 25%, preferably less than 10%, more preferably less than 5% of the reactivity exhibited toward a third molecule under a particular set of assay conditions, which includes the relative concentration and incubation of the molecules. Specific binding can be tested using a number of widely known methods, e.g, an immunohistochemical assay, an enzyme-linked immunosorbent assay (ELISA), a radioimmunoassay (RIA), or a western blot assay.

A "biological sample" refers to a sample from an animal subject including saliva, whole blood, serum, plasma, milk or other sample known to contain anti-SBV antibodies. A biological sample can be collected and stored by any convenient means that does not impact the ability of the sample to be tested in the method of the disclosure. Samples for use in the method of the disclosure may require preparation prior to use in the method. For example, samples may need to be precipitated, diluted, filtered or centrifuged to remove unwanted components or components may be added to the sample such as salts, detergents or additional proteins.

The term "solid support" or "solid phase" refers to a non-aqueous matrix to which a SBV or a Simbu group virus polypeptide can adhere by covalent or non-covalent binding. Examples of solid support include supports formed partially or entirely of glass (e.g., controlled pore glass), synthetic and natural polymers, polysaccharides (e.g., agarose), polyacrylamides, polystyrene, polyvinyl alcohols and silicones, magnetic particles, latex particles, chromatographic strips, microtiter polystyrene plates, or any other substances that will allow bound antigens to be washed or separated from unbound materials. In certain embodiments, depending on the application, the solid support can be the well of an assay plate or can be a purification column (e.g., an affinity chromatography column).

As used herein, the term "kit" or "assay kits" (e.g., articles of manufacture) refers to an assembly of useful compounds and other means like solid support plates or test stripes for detecting SBV infection or Simbu group virus in a mammalian sample, preferably ruminants. A kit therefore may include one or more devices and/or compositions of the present disclosure. In one particular example, such a kit includes the device having an immobilized SBV protein, fragment thereof or peptide, and comprising one or more capture antibody and other useful reagents like wash reagent, as well as detector reagent and positive and negative control reagents, if desired or appropriate. Other components such as buffers, controls, and the like, known to those of ordinary skill in art, may be included in such test kits. The relative amounts of the various reagents can be varied, to provide for concentrations in solution of the reagents that substantially optimize the sensitivity of the assay. Particularly, the reagents can be provided as dry powders, usually lyophilized, which on dissolution will provide for a reagent solution having the appropriate concentrations for combining with a sample. The present kit may further include instructions for carrying out one or more methods of the present disclosure, including instructions for using any device and/or composition of the present disclosure that is included with the kit.

In the following the particular aspects of the disclosure will be described in more detail.

In one aspect the disclosure is an isolated peptide comprising or consisting of 5 to 50 amino acids of the SBV nucleocapsid protein , preferably of 5 to 40, more preferably of 5 to 20 amino acids, even more preferably of 12 to 16 amino acids, even more preferably of 5 to 7 amino acids, preferably according to SEQ ID No. 2 to 138 (Table 1), or a fragment thereof or having at least 90% sequence identity with one of SEQ ID NO 2 - 138, preferably at least 95%, more preferably at least 98% sequence identity.

In a preferred embodiment of the disclosure the isolated peptide covers amino acid position 16 to 58 of Fig. 1 (SEQ ID NO: 1), preferably 16 to 32, 16 to 24, 18 to 35, 20 to 36, 35 to 50, 61 to 85, or 170 to 205, preferably 170 to 190 of the Nucleocapsid protein (Np) of SBV, more preferably 25 to 50, 61 to 85, 103 to 118 or 166 to 232, preferably 166 to 205 or 170 to 190 of the Nucleocapsid protein (Np) of SBV.

Additional preferred isolated peptides of the disclosure are described in the Examples, Figures and/or sequence listing. Such peptides can be applied in a suitable test format as single peptides or in combination of two, three, four, five, six or multiple peptides. Any such combination of peptides is within the gist of the disclosure and will result in positive test results with regard to selectivity and specificity.

Moreover, the peptides can vary due to the species variations. In a preferred embodiment the peptides will have at least 80%, preferably at least 90%, more preferably at least 95% and even more preferably at least 98% sequence identity to the peptides as described in the Examples, Figures, Tables and the sequence listing.

In another preferred embodiment the disclosure is a peptide mixture comprising at least two peptides according to the disclosure.

The peptides of the disclosure are characterized in that the peptide comprises or consists of an epitope specifically binding to an antibody produced by an animal infected with SBV or a Simbu serogroup virus.

In a preferred embodiment the isolated peptides according to the disclosure specifically bind to antibodies of cow and sheep origin.

The peptides of the disclosure either alone or in combination can advantageously be used for a peptide assay to identify SBV infection. The peptides will show superior results with respect to specificity and selectivity.

Particularly good results can be achieved with a peptide according to SEQ ID NO: 2 to 138.

Particularly preferred peptides of the disclosure are the following peptides of SEQ ID NO:1: NPEVGYVAFIGKYGQQLNFGVA, NPEVGYVAFIGKYGQQ, VGYVAFIGKYGQQLNF, VAFIGKYGQQLNFGVA, NPEVGYV, EVGYVAF, VAFIGKY, FIGKYGQ, GKYGQQL, YGQQLNF, QQLNFGV, QLNFGVA, NFGVA, LNFGV, QLNFG, QQLNF, GQQLN, YGQQL, KYGQQ, GKYGQ, IGKYG, FIGKY, AFIGK, VAFIG, YVAFI, GYVAF, VGYVA, EVGYV, PEVGY, NPEVG, LRQRYGTMTAEEWMTQKIT, GFSPAARTFLQQFGIN, LRQRYGT, QRYGTMT, YGTMTAE, TMTAEEW, TAEEWMT, EEWMTQK, WMTQKIT, GFSPAAR, SPAARTF, AARTFLQ, RTFLQQF, and LQQFGIN. One or more peptides can be used in a diagnostic test, or may be comprised in a longer sequence, preferred are at least two peptides, two, three of four peptides of this list.

A further preferred embodiment of the disclosure relates to an isolated peptide comprising 5 to 50 amino acids, preferably 5 to 40, more preferably 5 to 30, 35 or 40 and having at least one of SEQ ID NO: 2 to 138 contained therein.

The embodiments of the disclosure relate to one or more isolated peptides as described herein wherein the peptide comprises or consists of an epitope specifically binding to an antibody, preferably a polyclonal or monoclonal antibody, produced by an animal infected with SBV or a Simbu serogroup virus. The isolated peptide preferably binds specifically to antibodies of cow and sheep origin.

While all known detection formats can in principal be applied for the test, certain test formats will be preferred in view of practical aspects like ease of performing them and provision of test kits. In a preferred embodiment advantageously good results can be achieved in an indirect ELISA format known by the skilled person thus does not need to be described in detail here. More preferred is a single-step ELISA having the advantage that all reagents and the sample can be mixed in a one-step-process. Various preferred peptides useful in the tests according to the disclosure and the described formats are depicted in Fig. 3B and 4B. Further details as to the sequence and production of the peptides are depicted in the Examples, Tables, Figures and/or the sequences as depicted here and summarized in a sequence listing.

In another aspect the disclosure related to a method of making one or more peptides as described above.

In a preferred embodiment the disclosure relates to an in vitro method of identifying a non-human animal infected with SBV or a Simbu serogroup virus comprising the steps: i. contacting a sample with one or more peptides of the disclosure of SBV under conditions that a complex of an antibody specific against SBV or against a Simbu serogroup virus in the sample and the peptide can form;
ii. detecting the thus formed complex with an antibody specific against the antibody of the complex or a labeled peptide according to the disclosure wherein the detection of the complex is indicative of an infection with SBV or a Simbu serogroup virus.

In a preferred embodiment the method of identifying is an ELISA (enzyme linked immuno sorbent assay), preferably a multi-step and more preferably a single-step method.

The disclosure achieves a sensitivity of more than 95%, preferably more than 96%, even more preferred more than 97% and a specificity of more than 97%, preferably more than 98%, and even more preferably more than 99% with the peptides according to disclosure, preferably those of SEQ ID No. 2 to 138, more preferably those of SEQ ID NO: 2 to 75.

The samples that can be used in the methods, assays and test kits of the disclosure are from serum, plasma or milk, preferably of ruminants and are particularly useful to detect infection in cattle, sheep or goat.

In another aspect the disclosure relates to a kit for the detection of a SBV infection or a Simbu serogroup virus in a sample comprising:
i. one or more peptides of the disclosure as described above; and
ii. a means for detection of a complex formed between the peptide and an antibody specific against SBV or a Simbu serogroup virus

The kit may contain ready to use reagents and the test results are advantageously obtained within several hours, preferably within less than 6 hours, preferably less than 5 more preferably less than 4 hours.

The sample dilution factor is preferably less than or equal 50x, preferably 30x, preferably 20×, preferably 10× which advantageously avoids further dilution steps.

The kit preferably contains all ready to use reagents including coated plates, negative and positive controls, wash solution, sample diluent, conjugate, TMB and stop solutions.

The peptide antigen applied is preferably an antigen which is conserved among the Simbu group viruses and thus advantageously allows a pan-Simbu group detection of infection in a sample.

In preferred embodiments the solid phase of the test is coated with peptide antigen as described above. The peptide antigen can be chemically synthesized or expressed in E.coli. In the method and test kit any known and useful solid phase may be used, preferably MaxiSorp or PolySorp (Thermo Fisher Scientific) is used and coated by applying a coating buffer which has preferably a pH of 5, 7 or 9.5. The antigen is applied in a quantity of 0.1, 0.5, 1, 2, 3 or 4 ug/ml. It may be useful to apply a blocking solution known in the art but it is preferred to avoid a blocking solution.

As sample diluent may be used 0.14M NaCl, 2.7mM KCl, Kathon 0.03%, Tween20 0.1%. Another diluent useful according to the disclosure is 2% MgCl2, 6% Tween20 and 6% AO, 0.5% Casein sodium salt

The detection antibody is diluted 1:10000, preferably 1:20000.

The method steps will be applied as required and may vary depending to the particular reagents applied. In a preferred embodiment the conditions and method steps are as follows:
Sample (1:10) in sample diluent (MgCl2 2%, AO 6%, Tween20 6%, Casein 0.5%), 100 ul/well
Incubate 1 h, room temperature in humid chamber
3x wash (phosphate buffered saline with 0.1% Tween20)
Conjugate ready-to-use, 100 µl/well
Incubate 1 h, room temperature in humid chamber
3× wash (phosphate buffered saline with 0.1% Tween20)
TMB 100ul/well, incubate 10 mns, room temperature
Add stop solution (100 µl/well) and
Read out at 450nm

In particular preferred embodiments the sample diluent contains casein sodium salt in a concentration of between 0.1 to 0.55 %, preferably 0.1 %, more preferably 0.55 %. In preferred embodiments the sample diluent contains 0.5 % casein sodium salt and MgCl2, preferably the MgCl2 has a concentration of 2%.

In a preferred embodiment the method of detection, and preferably also the kit, is characterized by the inclusion of specific compounds, the use of particular dilutions of the capturing antigen and/or a particular amount and quality of capturing antigen coated onto the solid support used in the method of detection and the kit of the disclosure.

It could be shown that by way of the particular combination of preferably the purification conditions and/or the dilution conditions and/or the particular components included in the method of detection and kit an improved performance of the method of SBV detection and preferably of Simbu serogroup viruses could be achieved.

All members of this group could be detected with the method of the disclosure. Certain viruses are preferably detected: Schmallenberg virus, Shamonda virus, Douglas virus, Peaton virus, Akabane virus, Aino virus, Oropouche virus, Tinaroo virus, Shuni virus. Particularly preferred and particular good results were achieved with the following species: Peaton, Akabane, Aino, Tinaroo. In a particularly preferred embodiment the Simbu serogroup viruses of Bovidae, Cervidae and Camelidae are detected.

In a particularly preferred embodiment the dilution of the peptide of the disclosure was chosen to be in the coating solution in a concentration of 0.25 to 5 µg/ml, preferably 0.5 to 1 µg/ml. The inventive peptide preparation in its particular dilution proofed advantageous.

Moreover, the advantage of the test and kit according to the disclosure as described herein is not only evident from the positive test results achievable with the test as such. It is more so evident in comparison with test kits on the market as of today, i.e. August 2013. In particular the current disclosure and the method of detection in preferred embodiments is superior to SBV tests of the prior art, like the ID-Vet test and kit described above in the background section.

Another preferred embodiment uses in the method of detection and preferably in the kit casein. Any casein available in the market can be applied in a concentration which can be adapted according to the needs of the other compounds and in order to achieve good detection results in the test. Preferably the concentration in a solution of the test is 0.1 to 1 %, preferably 0.25 to 0.75 %, more preferably 0.5 %. In a preferred embodiment the casein is present in the sample diluents.

The coating step is preferably performed at pH 5 to 10, preferably about 5, 7 or 9.5.

The coating peptide as described in the specification and Examples, is used in amounts of 0.1, 0.5, 1, 2, or 4 µg/ml, preferably 1 µg/ml.

Another preferred embodiment uses alone or in addition aminoxid (AO). Preferably the concentration used is in a solution of the method in an amount of 3 to 10%, preferably 5 to 8%, more preferably 6%.

Another preferred embodiment of the method of detection and kit contains Tween, preferably a Tween20, or a comparable substance, preferably a detergent with comparable characteristics. This substance is contained in an amount of 0.05 to 0.5%, preferably 0.1 to 0.2 %.

In a particularly preferred embodiment the wash solution of the coating step contains preferably NaCl 0.14M, KCl 2.7 mM; Kathon 0.03%, Tween20 0.1%, sample diluent comprises MgCl2 2%, aminoxid (AO) 6%, Tween20 6% and 0.5% casein.

In a preferred embodiment, the anti-ruminant conjugate is used in a dilution of 1:10 000 to 1:30 000, preferably 1:20 000 in a conjugate stabilizing buffer as a ready to use format (RTU).

In another preferred embodiment the compounds as described above can be combined and will lead to particularly good test results. In such an embodiment the various compounds are contained in any of the solutions used for preparation of the kit compounds or during performing the test and may be contained in the kit according to the disclosure.

The inventors surprisingly achieved with one or a combination of the above described preferred embodiments very good results with regard to specificity and selectivity. It was not foreseeable that such good results could be obtained by using the particular antigen preparation in its particular dilution is particularly advantageous. More advantageous is the peptide antigen dilution in connection with the other additives like casein, aminoxid and/or Tween, preferably in the concentrations as described above.

The test preferably detects the Simbu serogroup viruses, it can preferably detect SBV in bovine, goat and sheep samples.

The cut off can be chosen according to the needs of sensitivity and specificity. Distribution is regarded to adapt it to fitness for purpose. This will depend on the needs of the test. ROC is used for analysis. The cut off is chosen according to standard procedures with regard to a positive and negative control and the respective calculations known to the person skilled in the art and literature for diagnostic test development.

In order to optimize the specificity the cut off is chosen preferably at 30% or more, 40% or more, 50% or more, or 60% or more.

In order to optimize the sensitivity the cut off is chosen preferably at 10% or more, 20% or more, 30% or more, or 40% or more.

An optimal cut off for the test according to the disclosure is preferably 40%, more preferably between 30 and 40%.

The advantage of the tests of the disclosure is that in preferred embodiments a specificity of more than 97%, preferably 98% and more preferably more than 99%, and a sensitivity of more than 94%, more preferably more than 95%, and more preferably more than 96% can be achieved. Particular embodiments achieve a specificity of about 99.8% and a sensitivity of about 95.8%. With regard to particular species the following specificities and sensitivities, respectively, can be achieved: cattle: 99.5% and 95.7%; sheep: 100% and 99%; goat: 100% and 99%.

Accordingly, the method and test kit will be particularly reliable for goat, sheep and cattle for the detection of SBV. In a preferred embodiment the good results will also apply to the Simbu serogroup. In particular good results can be achieved to detect Akabane, Peaton, Aino and Tinaroo virus.

In the following the disclosure will be illustrated by way of preferred examples which are not meant to be restrictive in any sense.

I. One aspect of the disclosure is described in the examples below.

### EXAMPLES

### Example 1

Development of a first generation screening test for detection of antibodies directed against Schmallenberg virus (Simbu serogroup ELISA)

In a first step the sequences of various Bunyaviruses were compared which is apparent from Table 1 below. Members of the Simbu serogroup showed a sequence similarity to the N protein of SBV of at least 78%.

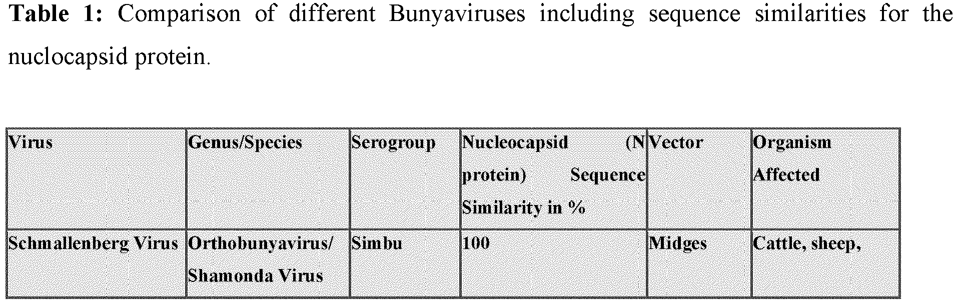

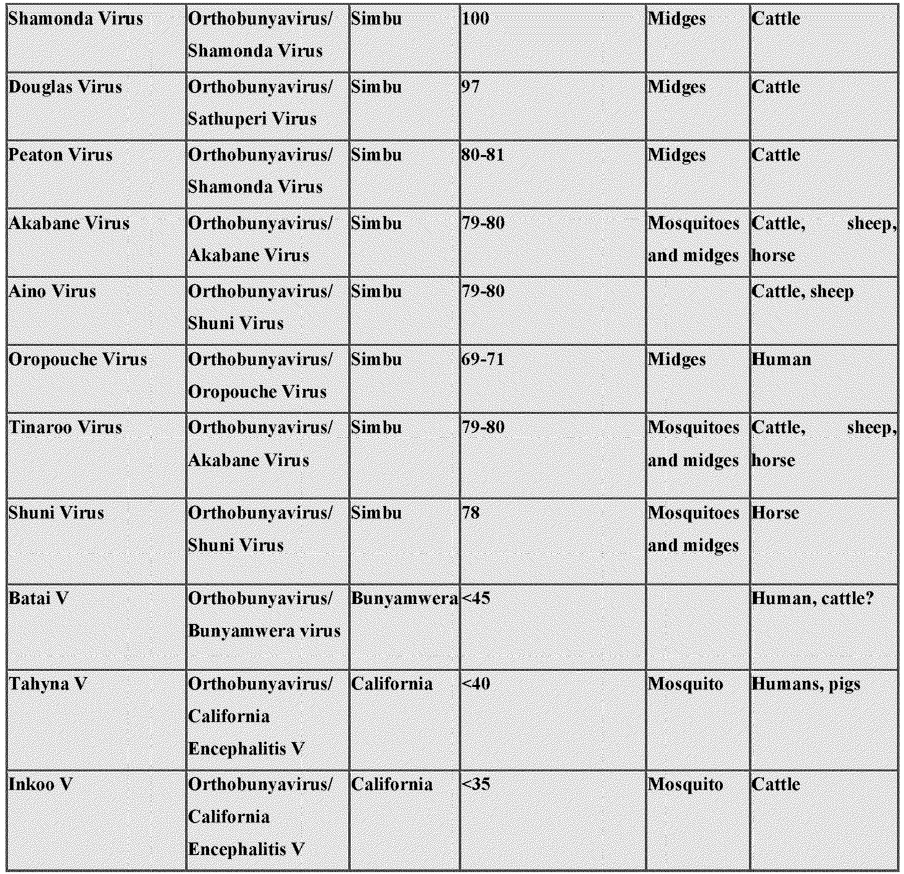

The sequence of the N protein clone is identical to the sequence SBV/1-846 presented in Table 2 below. The clone contains an additional His-tag.

### Expression, purification and biotinylation

The plasmid DNA was use to transform bacterial host cells and express the viral N (nucleocapsid) protein. A standard affinity purification system was established using Ni columns (His tagged N protein). The N protein shows a strong tendency to precipitate in buffered solutions, but only the buffer soluble fraction was used for further purification The binding and wash buffers contained 10 mM imidazole to prevent unspecific binding of contaminating E. coli proteins to the Ni column. The N protein was released from the column by increasing the imidazol concentration to 0.5 M in the elution buffer. Eluted proteins were used for direct coating experiments after removal of imidazol by dialysis against carbonate buffer pH 9.

Biotinylation of the N protein was done in alkaline carbonate buffer. Activated biotin was added and bound to the N protein. The biotinylation process was stopped by adding Tris buffer and free biotin was removed by dialysis against carbonate buffer without biotin. This material was used for coating experiments using plates pre-coated with streptavidin (plates from IDEXX CoE in Westbrook, ME, USA).

### Direct coating of protein to the plates:

The purified N protein (not biotinylated) was coated on Maxisorp plates using standard procedures (phosphate buffer, overnight incubation at 4°C). Plates were dried and antibody positive and negative samples were tested.

Coating Protocol:
- Dilution of purified N protein in phosphate buffer pH 7
- Addition of detergent to 15 µg/mlfinal concentration
- Incubation at room temperature for 20 min with shaking
- Further dilution of detergent treated antigen to 1 ― 4 µg/ml final concentration in phosphate buffer
- Coating of Maxisorp plates with 100 µl/ well
- Incubation over night at 4°C in a humid chamber
- Aspiration of coating solution and drying of plates
-

FIG. 1 depicts the results of the experimentation and is an example of antigen (purified N protein not biotinylated) titration in three different coating buffers (PBS pH 5; PBS pH 7; sodium carbonate buffer pH 9.3) tested with one positive (Pos. Bov FLI), one negative (NEG Bov. Sample) and no sample/blank.

### Example 2

### Streptavidin pre-coated plates and coupling of biotinylated N protein to the plates

Plates were pre-coated with streptavidin. Streptavidin plates were incubated with biotinylated N protein overnight, washed three times, and were not dried before the ELISA protocol was run. FIG. 6 shows an example of N protein biotinylated using a molar ratio of 2:1 (biotin:protein). Three positive samples were tested and one negative sample. The negative sample (ID 792) was used in the direct coat experiments as well (cf. Example 1). The positive samples are different from those in example 1. The ELISA protocol is identical with the protocol described for direct coat above of example 1.

FIG. 2 shows that in both cases there is a good differentiation between positive and negative samples over a range of various antigen concentrations.

### Example 3

### ELISA format I

In this format a direct coating of recombinant SBV nucleocapsid (N) protein is used. The coating protein can be either full length nucleocapsid protein or a truncated version according to SEQ ID No. 139 to 144. Other SBV proteins as depicted in the sequence listing can also be applied in the test.

The format is indirect and monophasic, and the sample type can be serum, plasma or milk.

The coated plates are incubated with serum, plasma or milk at room temperature. The samples can be applied as individual samples in single wells, in a dilution series and preferably as duplicates or triplicates. The sample dilution is preferably 1:10 or 1:20. After incubation for about 10 to 90 minutes, preferably about 30 to 60 minutes the plates are 3x washed preferably with PBS. For detection an anti-ruminant HRP-conjugated antibody is applied to the plate and incubated for about 10 to 90 minutes, preferably about 30 to 60 minutes. After a 3x wash substrate, preferably TMB is added for about 5 to 15 minutes, preferably 10 minutes, and the result is read out at 450nm. The plate is set up to include wells for samples and as well for positive and negative controls.

The recombinant nucleocapsid protein can be produced in E. coli or in a baculovirus system. Details as to the nucleocapsid protein used according to the disclosure are depicted in the sequence listing. Any of the nucleocapsid proteins listed therein can be applied in the test.

The coating is performed at a pH of about 5, 7 or 9.5 with or without blocking, preferably at pH 7. The coating protein is applied in amounts of o, 1, 2, or 4 µg/ml, preferably 1 µg/ml.

The wash solution contains preferably NaCl 0.14M, KCl 2.7 mM; Kathon 0.03%, Tween20 0.1%, sample diluent comprises MgCl2 2%, aminoxid (AO) 6%, Tween20 6% and 0.5% casein.

The anti-ruminant conjugate is used in a dilution of 1:10 000 to 1:30 000, preferably 1:20 000 in a conjugate stabilizing buffer as a ready to use format (RTU).

The test can detect SBV in bovine, goat and sheep samples.

### Example 4

### ELISA format II ― single step ELISA

The recombinant SBV protein, preferably nucleocapsid protein as described under ELISA format I is coupled to the solid support via streptavidin ― biotin. Preferably the streptavidin is coupled to the solid support and the SBV protein is coupled with biotin thus allowing coating to the solid support via the streptavidin-biotin complex that forms.

The solid support, e.g. a multiwall plate, is coated with streptavidin. The streptavidin coated plates are incubated in one single step with the biotinylated SBV protein (as described above; reference is made to the sequence listing of SBV proteins herein), the sample (preferably diluted as described above) and the anti-ruminant monoclonal antibody conjugated with HRP. The HRP antibody is an IgG preferably. After incubation for about 30 to 90 minutes, preferably 1 hour the plate is washed as described above and the read out is done at 450nm.

Microtiter plates are supplied precoated with streptavidin. The all-in-one buffer contains the biotinylated nucleocapsid protein, and the anti-IgG conjugate. Dilutions of the samples to be tested are incubated in the all-in-one buffer in wells of these plates. Any antibody specific for SBV binds to the antigen in the all-in-one buffer and forms an antigen/antibody/anti-IgG conjugate complex on the streptavidin-plate well surface. Unbound material is removed from the wells by washing. The TMB containing substrate is added to the wells. The degree of color that develops (optical density measured at 450 nm), is directly proportional to the amount of antibody specific for SBV present in the sample.

Results of the single step ELISA according to the disclosure in comparison to the IDvet test of the prior art are illustrated in Table 5

### Example 5

### Simbu serogroup recognition by ELISA (pan-Simbu group ELISA)

Preferred test formats according to the disclosure are capable of recognizing antibodies formed after infection with different members of the Simbu serogroup, i.e. the test is a pan-Simbu group test. See also Table 6.

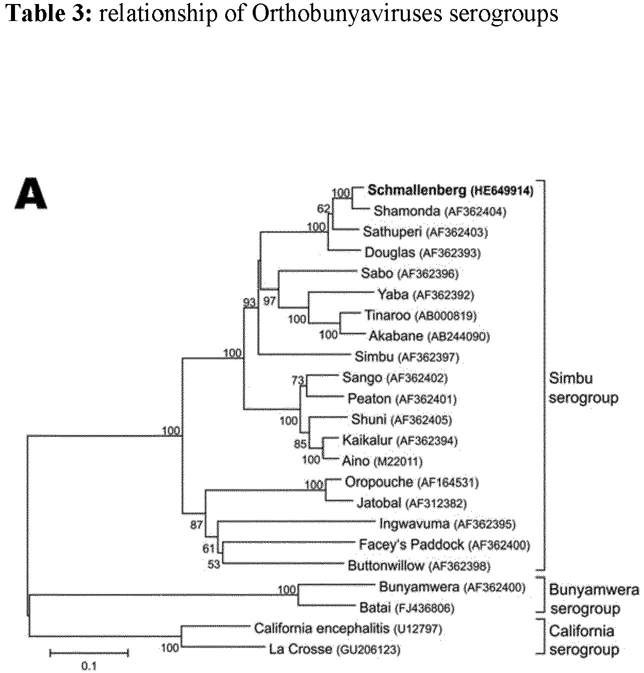

In a preferred embodiment the test is capable to detect the viruses of the Simbu serogroup, in a further preferred embodiment the Simbu and Bunyamwera, in a further preferred embodiment in addition the California serogroup, alternatively the Simbu and California serogroup in one test setup.

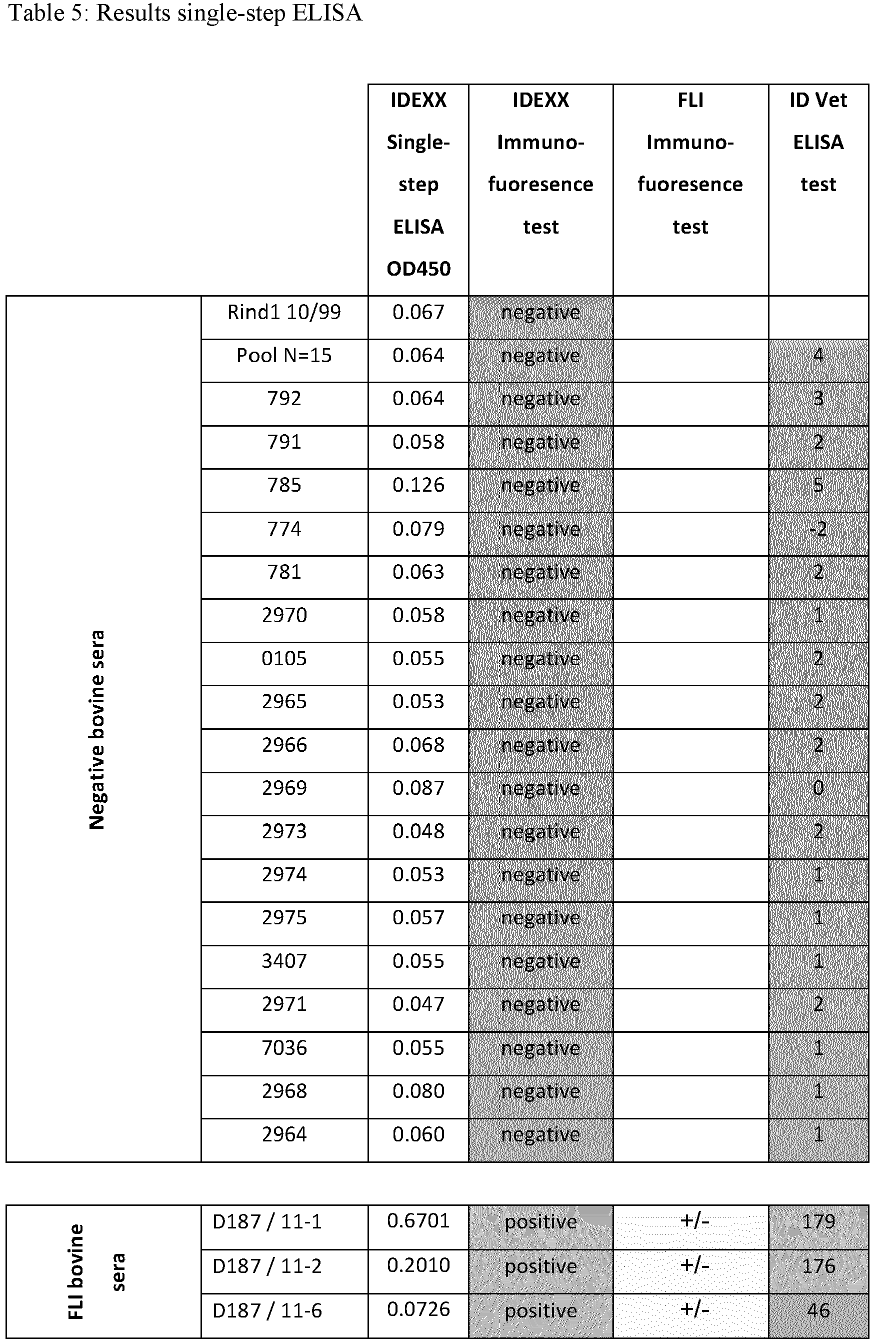

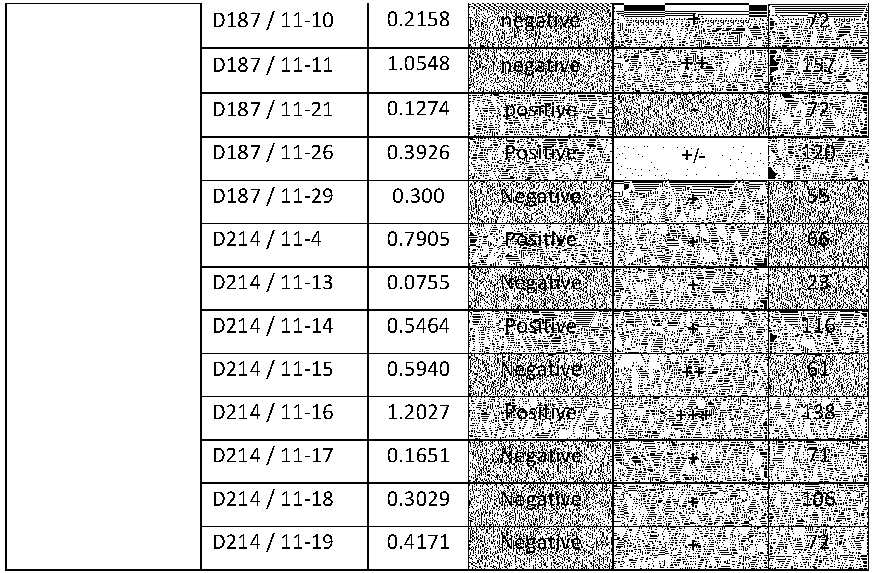

Table 5 describes a comparison of validated samples in the test according to the disclosure vis-à-vis the test marketed by IDvet.

The test results show the superior performance of the test and method according to the disclosure.

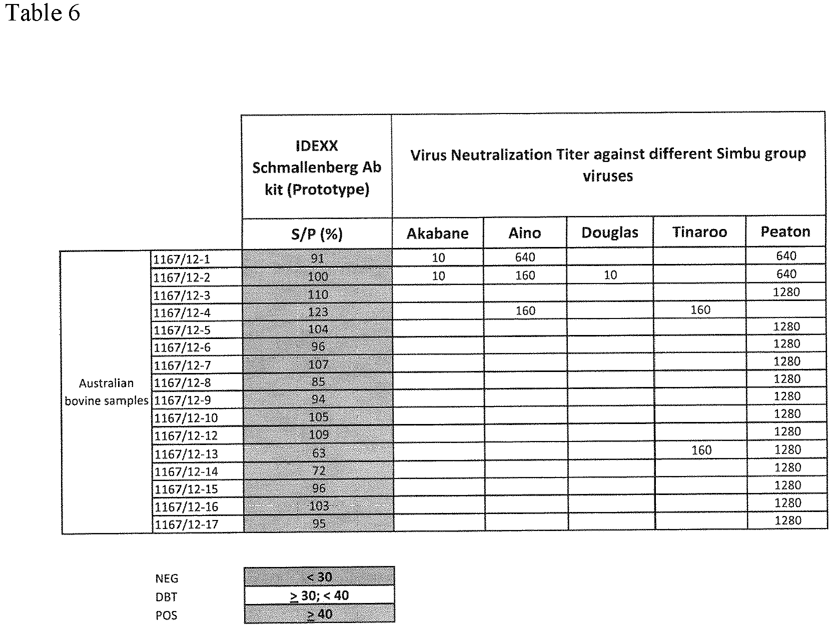

Table 6 shows validated bovine samples used in the test according to the disclosure. It confirms the detection of various Simbu serogroup viruses and thus confirms its usefulness as pan Simbu serogroup test.

II. Another aspect of the disclosure is described in the examples below.

### EXAMPLES

### Example 1

### Identification of peptides comprising epitope(s) useful for the establishment of a peptide diagnostic test

### PepScan mapping of epitopic regions on SBV Np viral proteins.

### Goal

An epitope mapping study was performed on SBV Np proteins. The goal was to identify the most immunogenic regions of these viral proteins for the 3 animal species relevant for SBV: cow, sheep and goat.

### Protocol

A PepScan approach was used, where overlapping peptides were synthesized all along the protein sequences. Synthesis of biotinylated peptides was done by NEP (New England Peptide, US). Biotinylated peptides were 16 amino acid long with 12 and 13 amino acid overlap for Np, respectively (see Table 1 to 4 for sequence information). Synthesis was successful for all requested peptides except for peptides number B10, D08, G05, H06 and H07 which were excluded from the analysis.

Biotinylated peptides were coated on streptavidin plates using a standard protocol. Characterized SBV positive and negative serum samples from the three SBV relevant animal species were tested for reactivity on each peptide. In addition, control reactivity with full length recombinant Np (rNP) was assessed on the same plate for each sample. Preliminary optimization studies led to the identification of experimental conditions allowing a good differentiation between positive and negative samples with limited background: sample dilution, sample diluent, and conjugate dilution. The ELISA format used was the classical indirect ELISA format. Briefly, after peptide coating during 1 h at room temperature (RT) streptavidin plates were washed. Bovine, ovine or caprine serum to be tested was subsequently added to each well and incubated for 1 h at RT. Plates were then washed and an anti-ruminant HRP-conjugated antibody was added and incubated for 1 h at RT. Plates were then again washed before the addition of TMB substrate. Color development was stopped after 10 min and optical densities (OD) were measured at 450 nm using a spectrophotometer.

Sera used as negative samples originated from the US. They tested clearly negative on IDEXX Schmallenberg antibody test (as marketed 2013) and showed also very low reactivity on streptavidin plates coated with full length rNP.

**Table 1. Sequence of peptides synthesized for SBV Np protein.**

| **Protein** | **Short ID** | **Peptide Sequence** | **QC** |
|---|---|---|---|
| Np | A01 | MSSQFIFEDVPQRNAA | Pass |
| Np | A02 | QFIFEDVPQRNAATFN | Pass |
| Np | A03 | FEDVPQRNAATFNPEV | Pass |
| Np | A04 | VPQRNAATFNPEVGYV | Pass |
| Np | A05 | RNAATFNPEVGYVAFI | Pass |
| Np | A06 | ATFNPEVGYVAFIGKY | Pass |
| Np | A07 | NPEVGYVAFIGKYGQQ | Pass |
| Np | A08 | VGYVAFIGKYGQQLNF | Pass |
| Np | A09 | VAFIGKYGQQLNFGVA | Pass |
| Np | A10 | IGKYGQQLNFGVARVF | Pass |
| Np | A11 | YGQQLNFGVARVFFLN | Pass |
| Np | A12 | QLNFGVARVFFLNQKK | Pass |
| Np | B01 | FGVARVFFLNQKKAKM | Pass |
| Np | B02 | ARVFFLNQKKAKMVLH | Pass |
| Np | B03 | FFLNQKKAKMVLHKTA | Pass |
| Np | B04 | NQKKAKMVLHKTAQPS | Pass |
| Np | B05 | KAKMVLHKTAQPSVDL | Pass |
| Np | B06 | MVLHKTAQPSVDLTFG | Pass |
| Np | B07 | HKTAQPSVDLTFGGVK | Pass |
| Np | B08 | AQPSVDLTFGGVKFTV | Pass |
| Np | B09 | SVDLTFGGVKFTVVNN | Pass |
| Np | B10 | LTFGGVKFTVVNNHFP | **Fail** |
| Np | B11 | GGVKFTVVNNHFPQYV | Pass |
| Np | B12 | KFTVVNNHFPQYVSNP | Pass |
| Np | C01 | VVNNHFPQYVSNPVPD | Pass |
| Np | C02 | NHFPQYVSNPVPDNAI | Pass |
| Np | C03 | PQYVSNPVPDNAITLH | Pass |
| Np | C04 | VSNPVPDNAITLHRMS | Pass |
| Np | C05 | PVPDNAITLHRMSGYL | Pass |
| Np | C06 | DNAITLHRMSGYLARW | Pass |
| Np | C07 | ITLHRMSGYLARWIAD | Pass |
| Np | C08 | HRMSGYLARWIADTCK | Pass |
| Np | C09 | SGYLARWIADTCKASV | Pass |
| Np | C10 | LARWIADTCKASVLKL | Pass |
| Np | C11 | WIADTCKASVLKLAEA | Pass |
| Np | C12 | DTCKASVLKLAEASAQ | Pass |
| Np | D01 | KASVLKLAEASAQIVM | Pass |
| Np | D02 | VLKLAEASAQIVMPLA | Pass |
| Np | D03 | LAEASAQIVMPLAEVK | Pass |
| Np | D04 | ASAQIVMPLAEVKGCT | Pass |
| Np | D05 | QIVMPLAEVKGCTWAD | Pass |
| Np | D06 | MPLAEVKGCTWADGYT | Pass |
| Np | D07 | AEVKGCTWADGYTMYL | Pass |
| Np | D08 | KGCTWADGYTMYLGFA | **Fail** |
| Np | D09 | TWADGYTMYLGFAPGA | Pass |
| Np | D10 | DGYTMYLGFAPGAEMF | Pass |
| Np | D11 | TMYLGFAPGAEMFLDA | Pass |
| Np | D12 | LGFAPGAEMFLDAFDF | Pass |
| Np | E01 | APGAEMFLDAFDFYPL | Pass |
| Np | E02 | AEMFLDAFDFYPLVIE | Pass |
| Np | E03 | FLDAFDFYPLVIEMHR | Pass |
| Np | E04 | AFDFYPLVIEMHRVLK | Pass |
| Np | E05 | FYPLVIEMHRVLKDNM | Pass |
| Np | E06 | LVIEMHRVLKDNMDVN | Pass |
| Np | E07 | EMHRVLKDNMDVNFMK | Pass |
| Np | E08 | RVLKDNMDVNFMKKVL | Pass |
| Np | E09 | KDNMDVNFMKKVLRQR | Pass |
| Np | E10 | MDVNFMKKVLRQRYGT | Pass |
| Np | E11 | NFMKKVLRQRYGTMTA | Pass |
| Np | E12 | KKVLRQRYGTMTAEEW | Pass |
| Np | F01 | LRQRYGTMTAEEWMTQ | Pass |
| Np | F02 | RYGTMTAEEWMTQKIT | Pass |
| Np | F03 | TMTAEEWMTQKITEIK | Pass |
| Np | F04 | AEEWMTQKITEIKAAF | Pass |
| Np | F05 | WMTQKITEIKAAFNSV | Pass |
| Np | F06 | QKITEIKAAFNSVGQL | Pass |
| Np | F07 | TEIKAAFNSVGQLAWA | Pass |
| Np | F08 | KAAFNSVGQLAWAKSG | Pass |
| Np | F09 | FNSVGQLAWAKSGFSP | Pass |
| Np | F10 | VGQLAWAKSGFSPAAR | Pass |
| Np | F11 | LAWAKSGFSPAARTFL | Pass |
| Np | F12 | AKSGFSPAARTFLQQF | Pass |
| Np | G01 | GFSPAARTFLQQFGIN | Pass |
| Np | G02 | PAARTFLQQFGINI | Pass |

### Results obtained with bovine samples

First focus was given on bovine sera. In first screening experiments a total of 4 SBV positive (colored lines) and 5 SBV negative bovine sera (black lines) were tested on all peptides as well as on the full length nucleoprotein (rNP). Results are summarized in Figure 1.

All positive samples show strong reactivity on the rNP full length control, whereas negative samples show very low reactivity, as expected. Several Np peptides or epitopic regions could be identified where a subset of positive samples bind much stronger than all negative samples (e.g. A09, A10, B03, B11, B12, C11, E10, E11, F04). There was no single epitopes identified showing reactivity with all bovine samples.

Selected peptides were then tested again with more sera using a stronger conjugate concentration for confirmation (Fig. 3B).

### Results obtained with ovine samples

Ovine sera were then tested on all Np peptides, following the same procedure (Figure 4). A total of n=6 SBV positive and 6 negative sheep sera were first screened for reactivity on all peptides. Several potential hits were identified in this first screen. Although no single hit could fit all positive samples, some hits showed strong reactivity for several positive sera as compared to negative sera (e.g. E09). The C-terminal region (starting at E07) showed a higher concentration of potential hits.

Peptides from the C-terminal region of Np starting at E07 were then tested again with more sera (Figure 4B, n=7 negative and n=9 SBV positive sera). Several positive sera showed confirmed strong reactivity on peptides E08-E09, E11, F01-F02 F04, and F12-G01 as compared to negative sera.

### Summary for identified epitopes

Several useful peptides with potential Np epitopes exhibiting diagnostic usefulness were identified in particular for cow and sheep (see Table 2 and 3, just below). The identified epitopic regions are also summarized in Fig. 6B.

In particular useful are peptides A09 to A11 and B03. This indicates that a peptide with amino acid position in the Np protein of position 25 to 58 will be particularly useful in a diagnostic test according to the disclosure.

Preferred is one peptide or a peptide mixture comprising several peptides with a length of between 7 and 20 amino acids covering, preferably comprising amino acid position 16 ― 32, 25 ― 45, 30 ― 50, 35 ― 55 or 38 ― 58 or fragments thereof containing 7, 8, 9, 10, 11, 12, 13, 14, 15, 16, 17, 18, 19 or 20 amino acids.

The sequences shown in Table 1 represent peptides having potential for usefulness in a diagnostic test to identify SBV infection in ungulate, preferably in cow and sheep, more preferably such peptides can be used to process and identify similarly cow and sheep samples. In another preferred embodiment a test applying such peptides can be used to identify an animal infected with a Simbu serogroup virus.

The peptides and fragments thereof particularly preferred are peptides A06 to A11, more preferably peptides A07, A08, A09. Peptides showing particularly good results in a test are the whole sequences or fragments of the sequences shown in Table 1, in particular those of peptides A07 and A08. Other preferred peptides are F01 to F12 and G01 to G02, more preferably F01, F03 and G01 of Table 1.

The skilled person will appreciate that the peptide as depicted in Table 1 show 16 amino acids, however, it will be sufficient to use shorter peptides as long as a stretch of at least 5 to 7 amino acids representing an epitope of a significant part of an epitope is contained, e.g. of A07 or A08, which exhibits a positive and specific binding characteristic to an antibody formed in an animal after e.g. SBV or Simbu serogroup virus infection.

Peptides particularly preferred and useful in a diagnostic peptide test as described herein are peptides comprising or consisting of the sequence NPEVGYVAFIGKYGQQLNFGVA, preferably NPEVGYVAFIGKYGQQ (SEQ ID NO: 76), VGYVAFIGKYGQQLNF (SEQ ID NO: 77), or VAFIGKYGQQLNFGVA (SEQ ID NO: 78), more preferably NPEVGYV (SEQ ID NO: 79), EVGYVAF (SEQ ID NO: 80), VAFIGKY (SEQ ID NO: 81), FIGKYGQ (SEQ ID NO: 82), GKYGQQL (SEQ ID NO: 83), YGQQLNF (SEQ ID NO: 84), QQLNFGV (SEQ ID NO: 85), or QLNFGVA (SEQ ID NO: 86), and even more preferably NFGVA (SEQ ID NO: 87), LNFGV (SEQ ID NO: 88), QLNFG (SEQ ID NO: 89), QQLNF (SEQ ID NO: 90), GQQLN (SEQ ID NO: 91), YGQQL (SEQ ID NO: 92), KYGQQ (SEQ ID NO: 93), GKYGQ (SEQ ID NO: 94), IGKYG (SEQ ID NO: 95), FIGKY (SEQ ID NO: 96), AFIGK (SEQ ID NO: 97), VAFIG (SEQ ID NO: 98), YVAFI (SEQ ID NO: 99), GYVAF (SEQ ID NO: 100), VGYVA (SEQ ID NO: 101), EVGYV (SEQ ID NO: 102), PEVGY (SEQ ID NO: 103), or NPEVG (SEQ ID NO: 104).

Peptides particularly preferred and useful in a diagnostic peptide test as described herein are peptides comprising or consisting of the sequence LRQRYGTMTAEEWMTQKIT and GFSPAARTFLQQFGIN, preferably fragments thereof, e.g. LRQRYGT (SEQ ID NO: 105), QRYGTMT (SEQ ID NO: 106), YGTMTAE (SEQ ID NO: 107), TMTAEEW (SEQ ID NO: 108), TAEEWMT (SEQ ID NO: 109), EEWMTQK (SEQ ID NO: 110), WMTQKIT (SEQ ID NO: 111), GFSPAAR (SEQ ID NO: 112), SPAARTF (SEQ ID NO: 113), AARTFLQ (SEQ ID NO: 114), or RTFLQQF (SEQ ID NO: 115), LQQFGIN (SEQ ID NO: 116).

Moreover, the inventors could identify some core motifs of the Np protein which represent amino acid sequences useful for identification of a virus infection in the sense of the application, e.g. SBV infection.

Such sequence motifs are preferably FFLN (Bov 43-46) (SEQ ID NO: 117) or VFFLN (Bov 42-46) (SEQ ID NO: 118) or FFLNQ (Bov 43-47) (SEQ ID NO: 119), MDVNPMKKVLRQR (Bov/Ov 172-184) (SEQ ID NO: 120), MKKVLRQR (Bov/Ov 177-184) (SEQ ID NO: 121), LRQR (Bov/Ov 181-184) (SEQ ID NO: 122) or LRQRYGTMTA (Bov/Ov 181-190) (SEQ ID NO: 123).

Peptides comprising or consisting of the above sequence motifs or amino acid sequences comprising said sequence motifs with less than 50 amino acids, preferably between 4 and 40, preferably with 4 to 25, preferably with 4 to 16 amino acids will be useful in the identification of SBV infection detection in a sample. Usual tests can be applies in order to show the advantageous usefulness for the detection of SBV infection or other Simbu group virus infection, most preferred SBV detection.

In another preferred embodiment the disclosure related to an isolated peptide comprising 5 to 50 amino acids and having at least one of SEQ ID No. 2 to 138 contained therein.

Other preferred amino acid sequences and epitopes or/and amino acid sequences comprising useful epitopes are depicted in the below Tables 2 and 3

**Table 2. Preferred cow epitope regions useful for a diagnostic test**

| **Peptide short ID** | **Np amino acid position** |
|---|---|
| A09 to A11 | 25-46 |
| B03 | 43-58 |
| B09 to B12 | 61-85 |
| C11 | 103-118 |
| E10 to E11 | 172-190 |
| F04 | 190-205 |

**Table 3. Preferred sheep epitope regions useful for a diagnostic test**

| **Peptide short ID** | **Np amino acid position** |
|---|---|
| E08 to E09 | 166-184 |
| E11 | 175-190 |
| F01 to F02 | 181-199 |
| F04 | 190-205 |
| F12 to G01 | 214-232 |

In addition to the Tables, Figure 6B summarizes identified epitopic consensus regions of useful amino acid stretches of the disclosure.

Additional useful sequences are depicted below which are further preferred embodiments useful for the detection of SBV in a peptide ELISA or comparable test formats, or in a preferred embodiment these peptides are useful in a pan-Simbu group test.

Nucleocapsid protein sequences recognized by bovine and ovine sera from SBV infected animals (preferred embodiments of the disclosure):
Bov 25-85 (SEQ ID NO: 124) 
VAFIGKYGQQLNFGVARVFFLNQKKAKMVLHKTAQPSVDLTFGGVKFTVVNNHFPQY
VSNP
 Bov 25-58 (SEQ ID NO: 125)
VAFIGKYGQQLNFGVARVFFLNQKKAKMVLHKTA
Bov 25-46 (SEQ ID NO: 126)
VAFIGKYGQQLNFGVARVFFLN
Bov 43-58 (SEQ ID NO: 127)
FFLNQKKAKMVLHKTA
Bov 61-85 (SEQ ID NO: 128)
SVDLTFGGVKFTVVNNHFPQYVSNP
Bov 103-118 (SEQ ID NO: 129)
WIADTCKASVLKLAEA
Bov+ Ov 166-232 (SEQ ID NO: 130) 
RVLKDNMDVNFMKKVLRQRYGTMTAEEWMTQKITEIKAAFNSVGQLAWAKSGFS
PAARTFLQQFGIN
Bov +Ov 166-205 (SEQ ID NO: 131)
RVLKDNMDVNFMKKVLRQRYGTMTAEEWMTQKITEIKAAF
Bov +Ov 166-184 (SEQ ID NO: 132)
RVLKDNMDVNFMKKVLRQR
Bov +Ov 172-205 (SEQ ID NO: 133)
MDVNFMKKVLRQRYGTMTAEEWMTQKITEIKAAF
Bov +Ov 172-190 (SEQ ID NO: 134)
MDVNFMKKVLRQRYGTMTA
Bov +Ov 175-190 (SEQ ID NO: 135)
NFMKKVLRQRYGTMTA
Bov +Ov 181-199 (SEQ ID NO: 136)
LRQRYGTMTAEEWMTQKIT
Bov +Ov 190-205 (SEQ ID NO: 137)
EEWMTQKITEIKAAF
Ov 214-232 (SEQ ID NO: 138)
KSGFSPAARTFLQQFGIN

The above peptides represent preferred embodiments of the disclosure and which sequences will show advantages results when applied alone or in combination in a test format as described herein. In particular preferred and showing good test results are the following peptides when applies in an immunoassay.

### Example 2

### Screening test for detection of antibodies directed against Schmallenberg virus (Simbu serogroup ELISA)

### Direct coating of peptides to the plates:

The purified peptides of the disclosure (not biotinylated) were coated on Maxisorp plates using standard procedures (phosphate buffer, overnight incubation at 4°C). Plates were dried and antibody positive and negative samples were tested.

Coating Protocol:
- Dilution of purified peptide(s) in phosphate buffer pH 7
- Addition of detergent to 15 µg/ml final concentration
- Incubation at room temperature for 20 min with shaking
- Further dilution of detergent treated antigen to 1 ― 4 µg/ml final concentration in phosphate buffer
- Coating of Maxisorp plates with 100 µl/ well
- Incubation over night at 4°C in a humid chamber
- Aspiration of coating solution and drying of plates

### Streptavidin pre-coated plates and coupling of biotinylated peptides(s) to the plates

Plates were pre-coated with streptavidin. Streptavidin plates were incubated with biotinylated peptide(s) of the disclosure overnight, washed three times, and were not dried before the ELISA protocol was run.. Three positive samples were tested and one negative sample. The negative sample (ID 792) was used in the direct coat experiments as well.

### Example 3

### ELISA format I

In this format a direct coating of peptide(s) of the disclosure is used. The coating peptide can be a single peptide or a combination of peptides according to the disclosure. The peptides are depicted in Table 1 and depicted in the sequence listing.

The format is indirect and monophasic, and the sample type can be serum, plasma or milk.

The coated plates are incubated with serum, plasma or milk at room temperature. The samples can be applied as individual samples in single wells, in a dilution series and preferably as duplicates or triplicates. The sample dilution is preferably 1:10 or 1:20. After incubation for about 10 to 90 minutes, preferably about 30 to 60 minutes the plates are 3x washed preferably with PBS. For detection an anti-ruminant HRP-conjugated antibody is applied to the plate and incubated for about 10 to 90 minutes, preferably about 30 to 60 minutes. After a 3x wash substrate, preferably TMB is added for about 5 to 15 minutes, preferably 10 minutes, and the result is read out at 450nm. The plate is set up to include wells for samples and as well for positive and negative controls.

The coating is performed at a pH of about 5, 7 or 9.5 with or without blocking, preferably at pH 7. The coating protein is applied in amounts of o, 1, 2, or 4 µg/ml, preferably 1 µg/ml.

The wash solution contains preferably NaCl 0.14M, KCl 2.7 mM; Kathon 0.03%, Tween20 0.1%, sample diluent comprises MgCl2 2%, aminoxid (AO) 6%, Tween20 6% and 0.5% casein.

The anti-ruminant conjugate is used in a dilution of 1:10 000 to 1:30 000, preferably 1:20 000 in a conjugate stabilizing buffer as a ready to use format (RTU).

The test can detect SBV in bovine and sheep samples.

### Example 4

### ELISA format II ― single step ELISA

The peptide(s) of the disclosure as described under ELISA format I is coupled to the solid support via streptavidin ― biotin. Preferably the streptavidin is coupled to the solid support and the peptide(s) is coupled with biotin thus allowing coating to the solid support via the streptavidin-biotin complex that forms.

The solid support, e.g. a multiwall plate, is coated with streptavidin. The streptavidin coated plates are incubated in one single step with the biotinylated peptide(s) (as described above; reference is made to the sequence listing of peptides herein), the sample (preferably diluted as described above) and the anti-ruminant monoclonal antibody conjugated with HRP. The HRP antibody is an IgG preferably. After incubation for about 30 to 90 minutes, preferably 1 hour the plate is washed as described above and the read out is done at 450nm.

Microtiter plates are supplied precoated with streptavidin. The all-in-one buffer contains the biotinylated peptide(s), and the anti-IgG conjugate. Dilutions of the samples to be tested are incubated in the all-in-one buffer in wells of these plates. Any antibody specific for SBV binds to the antigen in the all-in-one buffer and forms an antigen/antibody/anti-IgG conjugate complex on the streptavidin-plate well surface. Unbound material is removed from the wells by washing. The TMB containing substrate is added to the wells. The degree of color that develops (optical density measured at 450 nm), is directly proportional to the amount of antibody specific for SBV present in the sample.

### Example 5

### Simbu serogroup recognition by ELISA (pan-Simbu group ELISA)

Preferred test formats according to the disclosure are capable of recognizing antibodies formed after infection with different members of the Simbu serogroup, i.e. the test is a pan-Simbu group test.

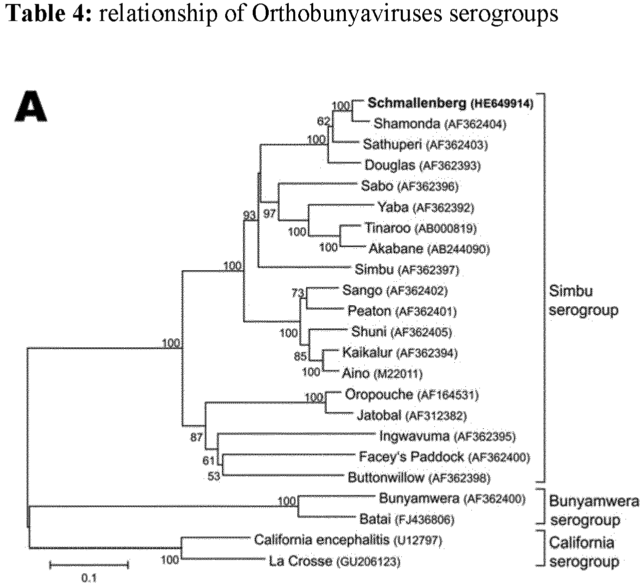

In a preferred embodiment the test is capable to detect the viruses of the Simbu serogroup, in a further preferred embodiment the Simbu and Bunyamwera, in a further preferred embodiment in addition the California serogroup, alternatively the Simbu and California serogroup in one test setup.

### Example 6

### Alternative peptide ELISA format

### Double recognition SBV ELISA Antibody Test and Test Kit

An alternative ELISA test format can be used as follows: The SBV double recognition antibody test can be applied to measure the antibody level to *SBV.* The test utilizes a synthetic SBV peptide according to the disclosure, preferably a peptide comprising the core motif sequences as described above or the other peptides pointed out as preferred embodiments of the disclosure, that mimics a *SBV Np* protein according to the disclosure as the diagnostic antigen.

A microtiter format can be devised in which the SBV peptide is coated on a 96-well microtiter plate. Prediluted test samples are incubated in the coated microtiter wells.

Antibodies specific to the SBV peptide (if present) will bind to the coated wells. Unbound components are washed away and a second SBV peptide which is coupled to horseradish peroxidase is incubated in the microtiter wells. The SBV peptide conjugate will bind to the second binding site of antibodies that had previously bound to the SBV peptide, thus forming specific immune complexes. Unbound components are washed away and an enzyme substrate (H₂O₂)/chromogen solution (tetramethylbenzidine, TMB) is added. Subsequent color development is proportional to the amount of specific antibody present in the sample.

The skilled person will appreciate that minor test setup modifications may be necessary depending on the SBV peptide applied in the test. These modifications are within the ordinary skill of the applicable skilled person.

**Table 5. Preferred amino acid/peptide sequences according to the disclosure**

| **SEQ ID NO:** | **Peptide sequence** |
|---|---|
| **SEQ ID NO:1** | **Np protein** |
| **SEQ ID NO: 2 - 13** | **A01 - A12 of Table 1** |
| **SEQ ID NO: 14 - 25** | **B01 ― B12 of Table 1** |
| **SEQ ID NO: 26 - 37** | **C01 ― C12 of Table 1** |
| **SEQ ID NO: 38 - 49** | **D01 ― D12 of Table 1** |
| **SEQ ID NO: 50 - 61** | **E01 ― E12 of Table 1** |
| **SEQ ID NO: 62 - 73** | **F01 ― F12 of Table 1** |
| **SEQ ID NO: 74 - 75** | **G01 ― G92 of Table 1** |
| **SEQ ID NO: 76 - 138** | **see above in text** |

### LITERATURE LIST

Hoffmann, B., Scheuch, M., Höper, D., Jungblut, R., Holsteg, M., Schirrmeier, H. (2012). Novel Orthobunyavirus in cattle, Europe, 2011. Emerg. Infect. Dis. 18, 469-472
Saeed, M. F., Li, L., Wang, H., Weaver, S. C., and Barret, A. D. T. (2001). Phylogeny of the Simbu serogroup of the genus Bunyavirus. J. Gen. Virol. 82, 2173-2181.
Goller, V. K., Höper, D., Schirrmeier, H., Mettenleiter, T. C., and Beer, M. (2012). Schmallenberg Virus as Possible Ancestor of Shamonda Virus. Emerg. Infect. Dis. 18,
Nichol, S. T., Beaty, B. J., Elliot, R. M., Goldbach, R., Plyusnin, A., Schmaljohn, C. S., and Tesh, R. B. (2005). Family Bunyaviridae. Edited by C. M. Fauquet, J. A. Mayo, J. Maniloff, U. Desselberger & L. A. Ball. Virus Taxonomy: Eighth Report of the International Committee on Taxonomy of Viruses. San Diego, CA, Elsevier, pp. 695-716.
Sambrook, J. and Russel, D. W. (2001). Molecular cloning: a laboratory manual. Third Edition, Cold Spring Habor Laboratory Press, Cold Spring Habor, New York
Eifant, S. A., Elliott, R. (2009). Mutational analysis of the Bunyamwera Orthobunyavirus nucleocapsid protein gene. J. Virol. 83, 11307-11317.
Savji, N., Palacios, G., Travassos da Rosa, A., Hutchinson, S., Celone, C., Hui, J., Briese, T., Calisher, C. H., Tesh, R. B., and Lipkin, W. I. (2011). Genomic and phylogenetic characterization of Leanyer virus, a novel orthobunyavirus isolated in northern Australia. J. Gen. Virol. 92, 1676-1687.

## Claims

1. An isolated peptide
selected from the group consisting of the sequences VAFIGKYGQQLNFGVA, IGKYGQQLNFGVARVF, FFLNQKKAKMVLHKTA, GGVKFTVVNNHFPQYV, KFTVVNNHFPQYVSNP, WIADTCKASVLKLAEA, RVLKDNMDVNFMKKVL, KDNMDVNFMKKVLRQR, MDVNFMKKVLRQRYGT, NFMKKVLRQRYGTMTA, LRQRYGTMTAEEWMTQ, RYGTMTAEEWMTQKIT, AEEWMTQKITEIKAAF, AKSGFSPAARTFLQQF, GFSPAARTFLQQFGIN.

2. An isolated peptide mixture comprising at least two peptides according to claim 1.

3. An in vitro method of identifying a non-human animal infected with Schmallenberg virus (SBV) comprising the steps:
i. contacting a sample with a peptide according to any of claims 1 to 2 under conditions that a complex of an antibody specific against SBV in the sample and the peptide can form;
ii. detecting the thus formed complex with an antibody specific against the antibody of the complex or a labeled peptide according to any of claims 1 to 2 wherein the detection of the complex is indicative of an infection with SBV, preferably wherein the method is an ELISA (enzyme linked immuno sorbent assay), preferably a multi-step or single-step method.

4. An in vitro use of one or more peptides according to any of claims 1 to 2 in a method for the detection of SBV infection.

5. A kit for the detection of a SBV infection in a sample comprising:
i. one or more peptides according to any of claims 1 to 2; and
ii. a means for detection of a complex formed between the peptide and an antibody specific against SBV.

6. An isolated peptide according to any of claims 1 ― 2 comprising an epitope useful for the specific identification of antibodies to SBV in a sample, wherein the epitope comprises at least 5 amino acids.

7. An in vitro method of identifying a non-human animal infected with SBV comprising the steps: i. contacting a sample with a peptide of SBV according to any of claims 1 ― 2 under conditions that a complex of an antibody specific against SBV in the sample and the peptide can form; ii. detecting the thus formed complex with an antibody specific against the antibody of the complex wherein the detection of the complex is indicative of an infection with SBV, preferably
wherein the method is an ELISA (enzyme linked immuno sorbent assay), preferably a multi-step or single-step method.

## Patentansprüche

1. Isoliertes Peptid,
ausgewählt aus der Gruppe, die aus den Sequenzen VAFIGKYGQQLNFGVA, IGKYGQQLNFGVARVF, FFLNQKKAKMVLHKTA, GGVKFTWNNHFPQYV, KFTWNNHFPQYVSNP, WIADTCKASVLKLAEA, RVLKDNMDVNFMKKVL, KDNMDVNFMKKVLRQR, MDVNFMKKVLRQRYGT, NFMKKVLRQRYGTMTA, LRQRYGTMTAEEWMTQ, RYGTMTAEEWMTQKIT, AEEWMTQKITEIKAAF, AKSGFSPAARTFLQQF und GFSPAARTFLQQFGIN besteht.

2. Mischung von isolierten Peptiden, die mindestens zwei Peptide nach Anspruch 1 umfasst.

3. In-vitro-Verfahren zur Identifizierung eines mit dem Schmallenberg-Virus (SBV) infizierten, nicht-menschlichen Tieres, umfassend die Schritte:
i. Inkontaktbringen einer Probe mit einem Peptid nach einem der Ansprüche 1 bis 2 unter Bedingungen, bei denen sich ein Komplex aus einem gegen SBV in der Probe spezifischen Antikörper und dem Peptid bilden kann;
ii. Nachweis des so gebildeten Komplexes mit einem Antikörper, der spezifisch gegen den Antikörper des Komplexes oder ein markiertes Peptid nach einem der Ansprüche 1 bis 2 ist, wobei der Nachweis des Komplexes auf eine Infektion mit SBV hinweist, vorzugsweise
wobei das Verfahren ein ELISA (Enzyme Linked Immuno Sorbent Assay), vorzugsweise ein mehrstufiges oder einstufiges Verfahren, ist.

4. In vitro-Verwendung eines oder mehrerer Peptide nach einem der Ansprüche 1 bis 2 in einem Verfahren zum Nachweis einer SBV-Infektion.

5. Kit für den Nachweis einer SBV-Infektion in einer Probe, umfassend:
i. ein oder mehrere Peptide nach einem der Ansprüche 1 bis 2, und
ii. ein Mittel zum Nachweis eines zwischen dem Peptid und einem gegen SBV spezifischen Antikörper gebildeten Komplexes.

6. Isoliertes Peptid nach einem der Ansprüche 1 bis 2, umfassend ein Epitop, das für die spezifische Identifizierung von Antikörpern gegen SBV in einer Probe nützlich ist, wobei das Epitop mindestens 5 Aminosäuren umfasst.

7. In-vitro-Verfahren zur Identifizierung eines mit SBV infizierten nicht-menschlichen Tieres, umfassend die Schritte: i. Inkontaktbringen einer Probe mit einem SBV-Peptid nach einem der Ansprüche 1 bis 2 unter Bedingungen, bei denen sich ein Komplex aus einem gegen SBV in der Probe spezifischen Antikörper und dem Peptid bilden kann; ii. Nachweis des so gebildeten Komplexes mit einem gegen den Antikörper des Komplexes spezifischen Antikörper, wobei der Nachweis des Komplexes auf eine Infektion mit SBV hinweist, vorzugsweise
wobei das Verfahren ein ELISA (Enzyme Linked Immuno Sorbent Assay), vorzugsweise ein mehrstufiges oder einstufiges Verfahren, ist.

## Revendications

1. Peptide isolé
choisi dans le groupe constitué par les séquences VAFIGKYGQQLNFGVA, IGKYGQQLNFGVARVF, FFLNQKAKMVLHKTA, GGVKFTWNNHFPQYV, KFTWNNHFPQYVSNP, WIADTCKASVLKLAEA, RVLKDNMDVNFMKKVL, KDNMDVNFMKKVLRQR, MDVNFMKKVLRQRYGT, NFMKKVLRQRYGTMTA, LRQRYGTMTAEEWMTQ, RYGTMTAEEWMTQKIT, AEEWMTQKITEIKAAF, AKSGFSPAARTFLQQF, GFSPAARTFLQQFGIN.

2. Mélange de peptides isolés comprenant au moins deux peptides selon la revendication 1.

3. Méthode in vitro d'identification d'un animal non humain infecté par le virus de Schmallenberg (SBV), comprenant les étapes consistant à :
i. mettre en contact un échantillon avec un peptide selon l'une quelconque des revendications 1 à 2 dans des conditions telles qu'un complexe d'un anticorps spécifique contre le SBV dans l'échantillon et le peptide puisse se former ;
ii. détecter le complexe ainsi formé avec un anticorps spécifique contre l'anticorps du complexe ou un peptide marqué selon l'une quelconque des revendications 1 à 2, la détection du complexe étant indicative d'une infection par le SBV, de préférence
dans laquelle la méthode est un ELISA (enzyme linked immuno sorbent assay), de préférence une méthode à plusieurs étapes ou à une seule étape.

4. Utilisation in vitro d'un ou plusieurs peptides selon l'une quelconque des revendications 1 à 2 dans une méthode de détection d'une infection par le SBV.

5. Kit pour la détection d'une infection par le SBV dans un échantillon, ledit kit comprenant :
i. un ou plusieurs peptides selon l'une quelconque des revendications 1 à 2 ; et
ii. un moyen de détection d'un complexe formé entre le peptide et un anticorps spécifique contre le SBV.

6. Peptide isolé selon l'une quelconque des revendications 1 à 2 comprenant un épitope utile pour l'identification spécifique d'anticorps contre le SBV dans un échantillon, l'épitope comprenant au moins 5 acides aminés.

7. Méthode in vitro d'identification d'un animal non humain infecté par le SBV comprenant les étapes consistant à : i. mettre en contact un échantillon avec un peptide du SBV selon l'une quelconque des revendications 1 à 2 dans des conditions telles qu'un complexe d'un anticorps spécifique contre le SBV dans l'échantillon et du peptide puisse se former ; ii. détecter le complexe ainsi formé avec un anticorps spécifique contre l'anticorps du complexe, la détection du complexe étant indicative d'une infection par le SBV, de préférence dans laquelle la méthode est un ELISA (enzyme linked immuno sorbent assay), de préférence une méthode à plusieurs étapes ou à une seule étape.
